# EUROPÄISCHE PATENTSCHRIFT

(11) **EP 4 011 319 B1**
(45) Veröffentlichungstag und Bekanntmachung des Hinweises auf die Patenterteilung: **16.10.2024**
(21) Anmeldenummer: 21213927.3
(22) Anmeldetag: 13.12.2021
(51) Int. Cl.: A61C 1/00, A61B 90/98, A61C 1/08

(54) **KUPPLUNGSELEMENT ZUM ANSCHLUSS EINES MEDIZINISCHEN ODER DENTALEN INSTRUMENTS AN EINE STEUER- ODER VERSORGUNGSEINHEIT**
COUPLING ELEMENT FOR CONNECTING A MEDICAL OR DENTAL INSTRUMENT TO A CONTROL OR SUPPLY UNIT
ÉLÉMENT DE COUPLAGE DESTINÉ À LA CONNEXION D'UN INSTRUMENT MÉDICAL OU DENTAIRE À UNE UNITÉ DE COMMANDE OU D'ALIMENTATION

(30) Priorität: 14.12.2020 US 202017121663
(43) Veröffentlichungstag der Anmeldung: 15.06.2022
(60) Teilanmeldung: 24199699.0
(73) Patentinhaber: W & H Dentalwerk Bürmoos GmbH, 5111 Bürmoos (AT)
(72) Erfinder: PRUCKNER, Christian, 1190 Wien (AT); SILBERER, Bernhard, 5152 Michaelbeuern (AT); EIBL, Johann, 5230 Mattighofen (AT)
(74) Vertreter: Benda, Ralf

(56) Entgegenhaltungen:
- EP-A1- 3 636 203
- US-A1- 2007 031 778
- US-A1- 2007 054 232
- US-A1- 2010 109 644

## Beschreibung

Die vorliegende Erfindung betrifft ein medizinisches oder dentales Instrumententeil mit einer mit elektrischer Energie betreibbaren Speichervorrichtung zur Speicherung von Identifikationsdaten und / oder Betriebsdaten und / oder Pflegedaten des Instrumententeils oder für das Instrumententeil.

Ein medizinisches oder dentales Instrumententeil mit einer mit elektrischer Energie betreibbaren Speichervorrichtung ist zum Beispiel aus der Patentanmeldung US 2003/0165794 A1 bekannt (auch als Patent US 6,899,538 veröffentlicht). Die Speichervorrichtung ist Teil einer Identifikationssignal-Ausgabevorrichtung zum aktiven Ausgeben von Identifikationssignalen, um das Instrumententeil zu identifizieren, so dass ein Schaltkreis einer Steuer-, Regel- oder Versorgungseinheit automatisch das Instrumententeil erkennt und entsprechend versorgt oder antreibt. Die Energieversorgung und Kommunikation zwischen der Speichervorrichtung und dem Antriebsschaltkreis erfolgt drahtgebunden über elektrische Leitungen, welche die Speichervorrichtung und die Steuer-, Regel- oder Versorgungseinheit elektrisch verbinden.

Die Patentanmeldung EP 3,636,203 A1 offenbart ein Instrumententeil und eine damit verbundene Steuer-, Regel- oder Versorgungseinheit. Die Steuer-, Regel- oder Versorgungseinheit umfasst eine als Konstantstromquelle ausgebildete Energiequelle, welche die Beleuchtungsvorrichtung mit einer konstanten Stromstärke versorgt. Des Weiteren weist das Instrumententeil eine Vorrichtung auf, welche die von der elektrischen Energiequelle übertragene elektrischer Energie mit konstanter elektrischer Stromstärke in elektrische Energie mit konstanter elektrischer Spannung wandelt, um damit eine Speichervorrichtung des Instrumententeils zu versorgen.

Der vorliegenden Erfindung liegt die Aufgabe zugrunde den Aufbau eines medizinischen oder dentalen Instrumententeils in Hinblick auf die drahtgebundene Energieversorgung und Kommunikationsverbindung/Datenübertragung zwischen der Speichervorrichtung und der Steuer-, Regel- oder Versorgungseinheit zu verbessern. Insbesondere soll der Aufbau des Instrumententeils einfach sein und die zur Energieversorgung und Kommunikationsverbindung zwischen der Speichervorrichtung und der Steuer-, Regel- oder Versorgungseinheit benötigten Bauteile wenig Platz einnehmen und eine zuverlässige Energieversorgung und Datenübertragung gewährleisten.

Diese Aufgabe wird gemäß der vorliegenden Erfindung durch ein medizinisches oder dentales Instrumententeil mit den Merkmalen des Anspruchs 1 und durch eine medizinische oder dentale Behandlungsvorrichtung mit den Merkmalen des Anspruchs 13 gelöst. Besonders vorteilhafte Ausführungsbeispiele sind in den Unteransprüchen angeführt.

Unter dem Begriff medizinisches oder dentales Instrumententeil' wird im Folgenden insbesondere verstanden: ein mit einer Hand haltbares medizinisches oder dentales Element, ein Handgriffelement, ein gerades Handstück, ein gewinkeltes oder gebogenes Handstück oder Winkelstück, ein Adapter, ein Kupplungselement, ein Antriebselement, insbesondere ein Luftmotor oder ein E-Motor, oder ein Versorgungsschlauch oder ein Teil der im Vorstehenden genannten Elemente.

Vorzugsweise umfasst das Instrumententeil ein medizinisches oder dentales Handstück oder zumindest einen Teil davon, das aufweist: Ein in Drehung versetzbares Antriebselement zum Antrieb eines mit dem Handstück lösbar verbindbaren Werkzeugs, eine Kupplungsvorrichtung zur Verbindung des Handstücks mit einer Steuer-, Regel- oder Versorgungseinheit, eine mit elektrischer Energie betreibbare Speichervorrichtung zur Speicherung von Identifikationsdaten und / oder Betriebsdaten und / oder Pflegedaten des Handstücks, eine mit elektrischer Energie betreibbare Beleuchtungsvorrichtung zur Abgabe von Strahlung auf eine Behandlungsstelle und zwei gemeinsame elektrische Leitungen.

Die Kupplungsvorrichtung zur Verbindung des Instrumententeils mit einer Steuer-, Regel- oder Versorgungseinheit ist insbesondere als lösbare Kupplungsvorrichtung ausgebildet, so dass das Instrumententeil von der Steuer-, Regel- oder Versorgungseinheit trennbar ist. Die Kupplungsvorrichtung ist zum Beispiel als Steckkupplung oder als Drehkupplung ausgebildet, so dass das Instrumententeil relativ zu der Steuer-, Regel- oder Versorgungseinheit drehbar ist, wenn das Instrumententeil mit dem Steuer-, Regel- oder Versorgungseinheit über die Kupplungsvorrichtung verbunden ist.

Es sind vorzugsweise zwei elektrische Kontakte an der Stirnfläche der Kupplungsvorrichtung angeordnet, wobei jeweils ein elektrischer Kontakt mit einer der zwei gemeinsamen elektrischen Leitungen verbunden ist. Für die dritte elektrische Leitung zum Übertragen von Identifikationsdaten und / oder Betriebsdaten und / oder Pflegedaten ist vorzugsweise in entsprechender Weise ein elektrischer Kontakt vorgesehen, der mit dieser dritten elektrischen Leitung für die Datenübertragung verbunden ist.

Vorzugsweise sind die elektrischen Kontakte lösbar mit der Steuer-, Regel- oder Versorgungseinheit verbunden. Die elektrischen Kontakt sind zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

Die an dem Instrumententeil vorgesehene oder mit dem Instrumententeil verbindbare Beleuchtungsvorrichtung umfasst vorzugsweise eine oder mehrere Lichtquellen, zum Beispiel optische Halbleiterelemente, insbesondere Leuchtdioden (LEDs). Vorzugsweise umfasst die Beleuchtungsvorrichtung mehrere Lichtquellen, wobei zumindest eine erste Lichtquelle (LED) eine erste Wellenlänge oder einen ersten Wellenlängenbereich und zumindest eine zweite Lichtquelle (LED) eine zweite Wellenlänge oder einen zweiten Wellenlängenbereich emittiert, die von der ersten Wellenlänge oder dem ersten Wellenlängenbereich unterschiedlich ist. Bevorzugt sind die erste Lichtquelle und die zweite Lichtquelle in unterschiedlichen elektrischen Stromrichtungen angeordnet. Besonders bevorzugt ist an dem Instrumententeil oder an einem mit dem Instrumententeil verbundenen oder verbindbaren Element, zum Beispiel der Steuer-, Regel- oder Versorgungseinheit, eine Schaltvorrichtung zum wahlweisen Betrieb der ersten Lichtquelle oder der zweiten Lichtquelle und / oder zur wahlweisen Emission von elektromagnetischer Strahlung durch die erste Lichtquelle oder durch die zweite Lichtquelle vorgesehen. Besonders bevorzugt steuert die Schaltvorrichtung den wahlweisen Betrieb der ersten Lichtquelle oder der zweiten Lichtquelle über die zumindest eine (gemeinsame) elektrische Leitung. Besonders bevorzugt ist die Schaltvorrichtung als elektrische Schaltvorrichtung ausgebildet, zum Beispiel als H-Brücke.

Wenn das Instrumententeil als medizinisches oder dentales Handstück ausgebildet ist, so wie dies im Vorstehenden beschrieben ist, so ist die Beleuchtungsvorrichtung vorzugsweise anschließend an einen oder an einem Kopfteil des Handstücks, in dem die Werkzeughalterung für das lösbar verbindbare Werkzeug gelagert ist, angeordnet. Insbesondere umfasst die Beleuchtungsvorrichtung mehrere optische Halbleiterelemente, die ringförmig um eine Werkzeugaufnahmeöffnung des Kopfteils positioniert sind.

Alternativ umfasst das Instrumententeil keine Beleuchtungsvorrichtung, sondern nur zumindest eine (gemeinsame) elektrische Leitung, die neben der Versorgung der Speichervorrichtung mit elektrischer Energie auch zur Versorgung der Beleuchtungsvorrichtung mit elektrischer Energie vorgesehen ist. Die Beleuchtungsvorrichtung ist in einem anderen Element angeordnet, das mit dem Instrumententeil mit der Speichervorrichtung derart verbindbar oder verbunden ist, dass die Beleuchtungsvorrichtung über die zumindest eine (gemeinsame) elektrische Leitung mit elektrischer Energie versorgbar ist.

Vorzugsweise ist eine der Beleuchtungsvorrichtung zugeordnete Vorrichtung zur Spannungsüberwachung vorgesehen, die ausgebildet ist, die der Beleuchtungsvorrichtung zugeführte elektrische Spannung zu überwachen. Die Spannungsüberwachung umfasst insbesondere einen Spannungsteiler, der die Eingangsspannung am Instrumententeil überwacht. Der Spannungsteiler weist zum Beispiel zwei elektrische Widerstände auf, die mit dem Mikrocontroller der Speichervorrichtung verbunden sind.

Es umfasst das Instrumententeil zumindest einen Sensor, der ausgebildet ist, einen Betriebszustand des Instrumententeils zu detektieren und ein, vorzugsweise elektrisches, Sensorsignal zu erzeugen.

Der Sensor weist zum Beispiel einen Temperatursensor zum Messen der Temperatur des Instrumententeils oder eines Abschnitts davon oder zumindest eines in dem Instrumententeil angeordneten Bauteils, insbesondere eines durch ein Antriebselement in Bewegung versetzbaren Bauteils, zum Beispiel eines Lagers, auf. Der Temperatursensor umfasst insbesondere einen elektrisch betreibbaren Temperatursensor, zum Beispiel einen Temperatursensor mit einem Material, dessen elektrischer Widerstand sich mit der Temperatur verändert (z.B. NTC-Temperatursensor), oder einen Infrarot-Temperatursensor.

Der Sensor weist alternativ zum Beispiel einen Drehzahlsensor zum Messen der Drehzahl eines Antriebselements des Instrumententeils oder eines damit verbindbaren Werkzeugs auf. Der Drehzahlsensor ist zum Beispiel als induktiver, kapazitiver oder optischer Drehzahlsensor ausgebildet.

Selbstverständlich kann der Sensor auch ausgebildet sein, andere Parameter zu messen, und zum Beispiel einen Sensor zum Messen einer Leistung, von Kraft oder Druck, eines Drehmoments, einer Lichtintensität oder Wellenlänge, etc. aufweisen.

Das Sensorsignal wird über die im Vorstehenden beschriebene separate Datenleitung an die Steuer-, Regel- oder Versorgungseinheit, insbesondere an einen darin angeordneten Mikrocontroller, geleitet. Der zumindest eine Sensor ist somit insbesondere derart über die separate Datenleitung mit der Steuer-, Regel- oder Versorgungseinheit verbunden, dass die Steuer-, Regel- oder Versorgungseinheit ein Sensorsignal empfängt und verarbeitet. Insbesondere ist die Steuer-, Regel- oder Versorgungseinheit ausgebildet, das Instrumententeil auf Basis eines von dem zumindest einem Sensor erhaltenen Sensorsignal zu steuern, zu regeln oder, zum Beispiel mit einem Betriebsmittel, zu versorgen.

Vorzugsweise ist in der Steuer-, Regel- oder Versorgungseinheit ein weiterer Mikrocontroller vorgesehen, der mit dem Mikrocontroller des Instrumententeils verbunden ist, wobei die gemeinsamen elektrischen Leitungen einen Teil dieser Verbindung bilden. Vorzugsweise ist auch die Energiequelle in der Steuer-, Regel- oder Versorgungseinheit angeordnet und insbesondere über die gemeinsamen elektrischen Leitungen mit der Beleuchtungsvorrichtung und der Speichervorrichtung elektrisch verbunden.

Vorzugsweise ist die Steuer-, Regel- oder Versorgungseinheit ausgebildet, das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals zu betreiben. Zum Beispiel ist die Steuer-, Regel- oder Versorgungseinheit ausgebildet, auf Basis dieser Daten einem Instrumententeil die auf dieses Instrumententeil abgestimmte Antriebsenergie und / oder zumindest ein bestimmtes Medium und / oder eine bestimmte Medienmenge zuzuleiten.

Vorzugsweise ist die Steuer-, Regel- oder Versorgungseinheit ausgebildet, auf das Instrumententeil zum Beispiel Daten bezüglich der Dauer des Betriebs des Instrumententeils, der Art seiner Verwendung und / oder der Art und / oder Dauer seiner Reinigung oder Pflege zu übertragen.

Vorzugsweise betreibt die Steuer-, Regel- oder Versorgungseinheit das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals, so wie dies im Vorstehenden beschrieben ist.

Erfindungsgemäß ist ein medizinisches oder dentales Instrumententeil vorgesehen, umfassend: eine Kupplungsvorrichtung, die das medizinische oder dentale Instrumententeil mit einem Versorgungsschlauch zum Anschluss des medizinischen oder dentalen Instrumententeils an eine Steuer-, Regel- oder Versorgungseinheit kuppelt; eine mit elektrischer Energie betreibbare medizinische oder dentale Instrumententeil-Steuerung; und drei elektrische Leitungen, umfassend eine erste gemeinsame elektrische Leitung, eine zweite gemeinsame elektrische Leitung und eine dritte elektrische Leitung. Die erste gemeinsame elektrische Leitung und die zweite gemeinsame elektrische Leitung sind mit der medizinischen oder dentalen Instrumententeil-Steuerung und mit einer elektromagnetische Strahlung emittierenden Vorrichtung verbunden, die an dem medizinischen oder dentalen Instrumententeil vorgesehen ist oder mit dem medizinischen oder dentalen Instrumententeil verbunden werden kann, und versorgen die elektromagnetische Strahlung emittierende Vorrichtung zum Betrieb der elektromagnetische Strahlung emittierenden Vorrichtung und die Steuereinheit des medizinischen oder dentalen Instrumententeils zum Betrieb der Steuereinheit des medizinischen oder dentalen Instrumententeils mit elektrischer Energie von einer gemeinsamen elektrischen Energiequelle. Die dritte elektrische Leitung der drei elektrischen Leitungen verbindet die medizinische oder dentale Instrumententeil-Steuerung mit der Steuer-, Regel- oder Versorgungseinheit. Das medizinische oder dentale Instrumententeil umfasst ferner zumindest einen Sensor, der zur Erzeugung eines Sensorsignals ausgebildet ist, wobei der zumindest eine Sensor mit der dritten elektrischen Leitung verbunden ist, so dass das Sensorsignal des Sensors über die dritte elektrische Leitung an die Steuer-, Regel- oder Versorgungseinheit übertragen werden kann.

Die medizinische oder dentale Instrumententeil-Steuerung umfasst vorzugsweise einen Mikrocontroller oder Mikrocomputer. Die medizinische oder dentale Instrumententeil-Steuerung umfasst vorzugsweise ein Speicherelement zur Speicherung von Identifikations- und/oder Betriebs- und/oder Pflegedaten des medizinischen oder dentalen Instrumententeils.

Die Vorrichtung zum Aussenden elektromagnetischer Strahlung sendet elektromagnetische Strahlung aus dem medizinischen oder dentalen Instrumententeil oder aus einer mit dem medizinischen oder dentalen Instrumententeil verbundenen Komponente aus. Die Vorrichtung zum Aussenden elektromagnetischer Strahlung sendet elektromagnetische Strahlung in Richtung eines Werkstücks des medizinischen oder dentalen Instrumententeils aus. Die von der elektromagnetischen Strahlungsemissionsvorrichtung emittierte elektromagnetische Strahlung umfasst zumindest eines der folgenden Elemente: sichtbare Strahlung oder sichtbares Licht, d. h. Strahlung mit zumindest einer Wellenlänge im Bereich zwischen 380 nm - 780 nm; weißes Licht; nicht sichtbare Strahlung, d. h. Strahlung mit Wellenlängen außerhalb des Bereichs von 380 nm - 780 nm; Laserlicht; IR-Strahlung; UV-Strahlung; Strahlung zur Diagnose; Strahlung zur Behandlung von Gewebe.

Die gemeinsame Stromquelle versorgt die Steuerung des medizinischen oder dentalen Instrumententeils und die elektromagnetische Strahlungsquelle mit Strom. Die gemeinsame Stromquelle versorgt vorzugsweise den zumindest einen Sensor des medizinischen oder dentalen Instrumententeils mit Strom, insbesondere wenn es sich bei dem zumindest einen Sensor um einen aktiven Sensor handelt, der zum Betrieb elektrische Energie benötigt. Die gemeinsame Stromquelle kann weitere Komponenten des medizinischen oder dentalen Instrumententeils oder mit dem medizinischen oder dentalen Instrumententeil gekoppelte Komponenten mit elektrischer Energie versorgen, z. B. ein Display oder ein Heizelement.

Die gemeinsame Stromquelle kann eine Konstantstromquelle, die elektrische Energie mit einer konstanten Stromstärke liefert, oder eine Konstantspannungsquelle, die elektrische Energie mit einer konstanten Spannung liefert, umfassen.

Die dritte elektrische Leitung der drei elektrischen Leitungen ist für die Übertragung von Daten zwischen der Steuer-, Regel- oder Versorgungseinheit und Komponenten des medizinischen oder dentalen Instrumententeils, insbesondere dem zumindest einen Sensor und/oder der medizinischen oder dentalen Instrumententeil-Steuerung vorgesehen. Vorzugsweise versorgen die erste gemeinsame elektrische Leitung und die zweite gemeinsame elektrische Leitung das medizinische oder dentale Instrumententeil nur mit elektrischer Energie, während die dritte elektrische Leitung nur Sensorsignale und/oder Daten überträgt, so dass vorteilhafterweise die Versorgung mit elektrischer Energie und die Übertragung von Sensorsignalen und/oder Daten auf unterschiedlichen, getrennten elektrischen Leitungen erfolgt, was vorzugsweise zumindest zu geringeren Interferenzen zwischen der zugeführten elektrischen Energie und den übertragenen Sensorsignalen und/oder Daten und damit zu einer stabileren Sensorsignal- und/oder Datenübertragung führt.

Die Übertragung von Daten über die dritte elektrische Leitung kann eine unidirektionale Übertragung umfassen, z.B. die Übertragung des Sensorsignals von dem zumindest einen Sensor zur Steuer-, Regel- oder Versorgungseinheit, insbesondere wenn der zumindest eine Sensor ein analoger Sensor ist. Die Datenübertragung kann eine bidirektionale Übertragung umfassen, z.B. die Übertragung von Daten zwischen dem Speicherelement der medizinischen oder dentalen Instrumententeil-Steuerung und der Steuer-, Regel- oder Versorgungseinheit und/oder wenn es sich bei dem zumindest einen Sensor um einen digitalen Sensor handelt.

Der zumindest eine Sensor ist mit der dritten elektrischen Leitung zur Übertragung des Sensorsignals oder eines vom Sensorsignal abgeleiteten Signals verbunden. Der zumindest eine Sensor kann direkt an die dritte elektrische Leitung angeschlossen werden, d. h. ohne ein Zwischenelement zwischen dem zumindest einen Sensor und der dritten elektrischen Leitung.

Alternativ ist der zumindest eine Sensor über die medizinische oder dentale Instrumententeil-Steuerung mit der dritten elektrischen Leitung verbunden. Vorzugsweise ist der zumindest eine Sensor elektrisch mit der medizinischen oder dentalen Instrumententeil-Steuerung verbunden, insbesondere über elektrische Leitungen. Dadurch wird das Sensorsignal des zumindest einen Sensors an die medizinische oder dentale Instrumententeil-Steuerung übertragen. Vorzugsweise wird das von dem zumindest einen Sensor erzeugte Sensorsignal oder ein von der medizinischen oder dentalen Instrumententeil-Steuerung verarbeitetes Sensorsignal oder ein von dem Sensorsignal abgeleitetes Signal von der medizinischen oder dentalen Instrumententeil-Steuerung und der dritten elektrischen Leitung zu der Steuer-, Regel- oder Versorgungseinheit übertragen.

Die dritte elektrische Leitung kann eine gemeinsame elektrische Leitung sein, die mit mehreren Komponenten eines medizinischen oder dentalen Instrumententeils oder mehreren medizinischen oder dentalen Instrumententeilen verbunden ist, z. B. mit dem zumindest einen Sensor und mit der medizinischen oder dentalen Instrumententeil-Steuerung.

Die medizinische oder dentale Instrumententeil-Steuerung versorgt den zumindest einen Sensor vorzugsweise mit elektrischer Energie, insbesondere wenn es sich bei dem zumindest einen Sensor um einen aktiven Sensor handelt, der zum Betrieb elektrische Energie benötigt. Besonders bevorzugt ist die medizinische oder dentale Instrumententeil-Steuerung ausgebildet, die über die erste gemeinsame elektrische Leitung und die zweite gemeinsame elektrische Leitung von der gemeinsamen elektrischen Energiequelle empfangene elektrische Energie zu verarbeiten und die verarbeitete elektrische Energie dem zumindest einen Sensor zuzuführen, so dass der zumindest eine Sensor mit der für ihn erforderlichen elektrischen Energie versorgt wird. Die medizinische oder dentale Instrumententeil-Steuerung versorgt den zumindest einen Sensor über elektrische Leitungen mit elektrischer Energie, wobei zumindest eine dieser elektrischen Leitungen zur Energieversorgung insbesondere auch zur Übertragung des Sensorsignals an die medizinische oder dentale Instrumententeil-Steuerung genutzt werden kann.

Alternativ ist der zumindest eine Sensor direkt (d.h. ohne dass die medizinische oder dentale Instrumententeil-Steuerung zwischen dem zumindest einen Sensor und der ersten und zweiten gemeinsamen elektrischen Leitung eingefügt ist) über die erste und zweite gemeinsame elektrische Leitung mit der gemeinsamen elektrischen Energiequelle verbunden, um elektrische Energie von der gemeinsamen elektrischen Energiequelle zu erhalten. Vorzugsweise ist dem zumindest einen Sensor (nur) eine Vorrichtung zur Aufbereitung der von der gemeinsamen Stromquelle gelieferten elektrischen Energie zugeordnet, um den zumindest einen Sensor mit der von ihm benötigten elektrischen Energie zu versorgen. Die Einrichtung zur Aufbereitung der elektrischen Leistung umfasst beispielsweise einen DC/DC-Wandler.

Vorzugsweise ist die medizinische oder dentale Instrumententeil-Steuerung so ausgebildet, dass sie das von dem zumindest einen Sensor erzeugte Sensorsignal entweder speichert oder verarbeitet.

Die Verarbeitung des Sensorsignals kann vorzugsweise die Umwandlung des von dem zumindest einen Sensor erzeugten Sensorsignals umfassen. Handelt es sich bei dem Sensorsignal beispielsweise um ein analoges Sensorsignal, ist die medizinische oder dentale Instrumententeil-Steuerung ausgebildet, das analoge Sensorsignal in ein digitales Sensorsignal umzuwandeln.

Die Verarbeitung des Sensorsignals kann vorzugsweise die Filterung des von dem zumindest einen Sensor erzeugten Sensorsignals umfassen. Die Verarbeitung des Sensorsignals kann vorzugsweise die Verringerung des Rauschens des von dem zumindest einen Sensor erzeugten Sensorsignals umfassen. Die Verarbeitung des Sensorsignals kann vorzugsweise die Verstärkung des von dem zumindest einen Sensor erzeugten Sensorsignals umfassen.

Die medizinische oder dentale Instrumententeil-Steuerung ist vorzugsweise dazu ausgebildet, das aufbereitete Sensorsignal zu speichern und/oder das aufbereitete Sensorsignal, insbesondere das umgewandelte digitale Sensorsignal, über die dritte elektrische Leitung der drei elektrischen Leitungen an die Steuer-, Regel- oder Versorgungseinheit zu senden.

Die medizinische oder dentale Instrumententeil-Steuerung ist vorzugsweise so ausgebildet, dass sie zumindest eine Komponente des medizinischen oder dentalen Instruments auf der Grundlage des von dem zumindest einen Sensor empfangenen Sensorsignals betreibt oder regelt oder steuert. Besonders bevorzugt speichert und/oder verarbeitet die medizinische oder dentale Instrumententeil-Steuerung das Sensorsignal wie oben beschrieben, bevor sie die zumindest eine Komponente des medizinischen oder dentalen Instruments basierend auf dem Sensorsignal betreibt oder regelt oder steuert.

Beispielsweise kann die medizinische oder dentale Instrumententeil-Steuerung auf der Grundlage des Sensorsignals den Antrieb oder die Bewegung einer Komponente des medizinischen oder dentalen Instrumententeils starten oder stoppen oder beschleunigen oder abbremsen. Alternativ oder zusätzlich kann die medizinische oder dentale Instrumententeil-Steuerung auf der Grundlage des Sensorsignals die Emission von elektromagnetischer Strahlung der Vorrichtung zum Aussenden elektromagnetischer Strahlung starten oder stoppen und/oder die Strahlungsintensität oder die Wellenlänge der von der Vorrichtung zum Aussenden elektromagnetischer Strahlung zu emittierenden Strahlung verändern. Alternativ oder zusätzlich kann die medizinische oder dentale Instrumententeil-Steuerung auf der Grundlage des Sensorsignals die Abgabe eines Mediums, einer Flüssigkeit, Wasser, Luft, eines Medikaments oder eines diagnostischen Mittels starten oder stoppen. Alternativ oder zusätzlich kann die medizinische oder dentale Instrumententeil-Steuerung auf der Grundlage des Sensorsignals die Bestimmung, Messung, Aufzeichnung eines Wertes, insbesondere durch einen Sensor, oder die Aufnahme eines Bildes, insbesondere durch einen Sensor oder eine Kamera, starten oder stoppen.

Vorzugsweise ist das Speicherelement mit der dritten elektrischen Leitung verbunden, so dass die Identifikations- und/oder Betriebs- und/oder Pflegedaten des medizinischen oder dentalen Instrumententeils über die dritte elektrische Leitung zwischen der Steuer-, Regel- oder Versorgungseinheit und dem Speicherelement übertragen werden können. Die dritte elektrische Leitung dient somit zur Übertragung des Sensorsignals des zumindest einen Sensors und von Identifikations- und/oder Betriebs- und/oder Pflegedaten, was vorteilhaft die Anzahl der elektrischen Leitungen im medizinischen oder dentalen Instrumententeil reduziert.

Das medizinische oder dentale Instrumententeil umfasst vorzugsweise eine Vorrichtung zur Aufbereitung elektrischer Energie, die ausgebildet ist, dass sie die von der gemeinsamen elektrischen Energiequelle über die erste oder zweite gemeinsame elektrische Leitung an das medizinische oder dentale Instrumententeil gelieferte elektrische Energie aufbereitet.

Insbesondere ist die Vorrichtung zur Aufbereitung elektrischer Energie so ausgebildet, dass sie elektrische Energie mit konstanter Spannung oder Gleichstrom bereitstellt. Alternativ ist die Vorrichtung zur Aufbereitung von elektrischer Energie so ausgebildet, dass sie elektrische Energie mit konstanter Stromstärke oder Wechselstrom bereitstellt.

Die Vorrichtung zur Aufbereitung elektrischer Energie kann aufbereitete elektrische Energie, vorzugsweise elektrische Energie mit konstanter Spannung oder Gleichstrom, für mehrere Elemente des mit elektrischer Energie versorgten medizinischen oder dentalen Instrumententeils bereitstellen, zum Beispiel für zumindest zwei oder alle der folgenden Elemente: der medizinischen oder dentalen Instrumententeil-Steuerung; zumindest einem Sensor; der elektromagnetische Strahlung emittierenden Vorrichtung; anderen Komponenten.

Alternativ ist die Vorrichtung zur Aufbereitung elektrischer Energie einem einzelnen Element des mit elektrischer Energie versorgten medizinischen oder dentalen Instrumententeils zugeordnet und kann aufbereitete elektrische Energie, vorzugsweise elektrische Energie mit konstanter Spannung oder Gleichstrom, nur für dieses einzelne Element bereitstellen, dem sie zugeordnet ist. Beispielsweise ist die Vorrichtung zur Aufbereitung elektrischer Energie nur einem der folgenden Elemente des medizinischen oder dentalen Instrumententeils zugeordnet und versorgt dieses mit elektrischer Energie: der medizinischen oder dentalen Instrumententeil-Steuerung; zumindest einem Sensor; der elektromagnetische Strahlung emittierenden Vorrichtung; einer weiteren Komponente.

Die Vorrichtung zur Aufbereitung elektrischer Energie kann einen AC/DC-Wandler oder Gleichrichter umfassen. Der AC/DC-Wandler ist beispielsweise so ausgebildet, dass er elektrische Energie mit konstanter Stromstärke, die von der gemeinsamen elektrischen Energiequelle mit einer Konstantstromquelle über die erste und zweite gemeinsame elektrische Leitung geliefert wird, in elektrische Energie mit konstanter Spannung umwandelt. Vorzugsweise kann der AC/DC-Wandler oder Gleichrichter durch elektronische Bauteile oder durch eine elektronische oder integrierte Schaltung gebildet sein. Besonders bevorzugt ist der AC/DC-Wandler oder Gleichrichter so ausgebildet oder angeordnet, dass er Gleichstrom für alle elektrischen Komponenten des medizinischen oder dentalen Instrumententeils bereitstellt, insbesondere für die medizinische oder dentale Instrumententeil-Steuerung, den zumindest einen Sensor und die elektromagnetische Strahlung emittierende Vorrichtung.

Die Vorrichtung zur Aufbereitung elektrischer Energie kann einen DC/AC-Wandler oder Wechselrichter umfassen. Der DC/AC-Wandler ist beispielsweise so ausgebildet, dass er elektrische Energie mit konstanter Spannung, die von der gemeinsamen elektrischen Energiequelle mit konstanter Spannungsquelle über die erste und zweite gemeinsame elektrische Leitung geliefert wird, in elektrische Energie mit konstanter Stromstärke umwandelt. Vorzugsweise kann der DC/AC-Wandler oder Wechselrichter durch elektronische Bauteile oder durch eine elektronische oder integrierte Schaltung gebildet sein.

Ein AC/DC-Wandler ist vorzugsweise nur der elektromagnetische Strahlung aussendenden Vorrichtung zugeordnet und versorgt nur die elektromagnetische Strahlung emittierende Vorrichtung mit einer konstanten Stromstärke. Besonders bevorzugt ist der nur der elektromagnetischen Strahlungsquelle zugeordnete AC/DC-Wandler Teil einer nur der elektromagnetischen Strahlungsquelle zugeordneten Konstantstromquelle.

Die Vorrichtung zur Aufbereitung der elektrischen Energie umfasst erfindungsgemäß einen DC/DC-Wandler. Der DC/DC-Wandler ist mehreren mit elektrischer Energie versorgten Elementen des medizinischen oder dentalen Instrumententeils zugeordnet und/oder versorgt diese mit elektrischer Energie, beispielsweise zumindest zwei oder allen der folgenden Elemente: der medizinischen oder dentalen Instrumententeil-Steuerung; dem zumindest einen Sensor; der elektromagnetische Strahlung emittierenden Vorrichtung; weiteren Komponenten. Zusätzlich kann ein DC/DC-Wandler vorgesehen sein, der nur einem einzigen mit elektrischer Energie versorgten Element des medizinischen oder dentalen Instrumententeils zugeordnet ist und/oder elektrische Energie für dieses aufbereitet, beispielsweise nur für eines der folgenden Elemente: der medizinischen oder dentalen Instrumententeil-Steuerung; dem zumindest einen Sensor; der elektromagnetische Strahlung emittierenden Vorrichtung; einer weiteren Komponente. Vorzugsweise kann der DC/DC-Wandler durch elektronische Bauteile oder durch eine elektronische oder integrierte Schaltung gebildet sein.

Die Vorrichtung zur Aufbereitung elektrischer Energie kann einen Filter umfassen, um beispielsweise Übertragungsstörungen der elektrischen Energie zu kompensieren, die von der gemeinsamen elektrischen Energiequelle über die erste und zweite gemeinsame elektrische Leitung geliefert wird. Auch hier kann der Filter entweder nur einem einzelnen mit elektrischer Energie versorgten Element des medizinischen oder dentalen Instrumententeils zugeordnet sein und die elektrische Energie nur für dieses einzelne Element filtern, dem er zugeordnet ist, oder mehreren oder allen elektrischen Elementen des medizinischen oder dentalen Instrumententeils und die elektrische Energie für diese mehreren oder alle Elemente filtern, wie oben beschrieben.

Das medizinische oder dentale Instrumententeil umfasst vorzugsweise eine elektrische Konstantstromquelle, die nur der elektromagnetische Strahlung emittierenden Vorrichtung zugeordnet ist und die ausgebildet ist, nur die elektromagnetische Strahlung emittierende Vorrichtung mit elektrischer Energie mit einer konstanten Stromstärke zu versorgen. Die elektrische Konstantstromquelle ist über die erste und zweite gemeinsame elektrische Leitung mit der gemeinsamen elektrischen Stromquelle verbunden, um von der gemeinsamen elektrischen Stromquelle elektrische Energie zum Betrieb der Vorrichtung zur Emission elektromagnetischer Strahlung zu erhalten. Vorzugsweise ist die gemeinsame elektrische Stromquelle eine Konstantspannungsquelle oder umfasst eine solche. Vorzugsweise erhält die elektrische Konstantstromquelle elektrische Energie mit konstanter Spannung oder Gleichstrom.

Vorzugsweise kann die elektrische Konstantstromquelle aus elektronischen Komponenten oder einer integrierten Schaltung bestehen. Vorzugsweise ist die integrierte Schaltung so ausgebildet, dass sie elektrische Leistung mit konstanter Spannung oder Gleichstrom in elektrische Leistung mit konstanter Stromstärke oder Wechselstrom umwandelt. Vorzugsweise kann die elektrische Konstantstromquelle einen Shunt-Widerstand umfassen.

Die medizinische oder dentale Instrumententeil-Steuerung ist vorzugsweise so ausgebildet, dass sie die elektromagnetische Strahlungsabgabe der elektromagnetische Strahlung emittierenden Vorrichtung steuert. Besonders bevorzugt ist die medizinische oder dentale Instrumententeil-Steuerung ausgebildet, die Intensität der von der elektromagnetische Strahlung emittierenden Vorrichtung emittierten elektromagnetischen Strahlung zu steuern. Besonders bevorzugt ist die medizinische oder dentale Instrumententeil-Steuerung ausgebildet, die elektromagnetische Strahlung emittierenden Vorrichtung zu dimmen, insbesondere um das von der elektromagnetische Strahlung emittierenden Vorrichtung emittierte sichtbare Licht zu dimmen. Besonders bevorzugt ist die medizinische oder dentale Instrumententeil-Steuerung ausgebildet, das Nachleuchten der elektromagnetische Strahlung emittierenden Vorrichtung, insbesondere das Nachleuchten des von der elektromagnetische Strahlung emittierenden Vorrichtung emittierten sichtbaren Lichts, zu steuern, so dass elektromagnetische Strahlung von der elektromagnetische Strahlung emittierenden Vorrichtung emittiert wird, wenn ein anderer Betrieb der medizinischen oder dentalen Instrumententeil-Steuerung gestoppt wird, z.B. der Antrieb eines mit der medizinischen oder dentalen Instrumententeil-Steuerung verbundenen Werkzeugs und/oder die Abgabe eines Fluids.

Zur Steuerung der elektromagnetischen Strahlungsemission der elektromagnetische Strahlung emittierenden Vorrichtung ist die medizinische oder dentale Instrumententeil-Steuerung vorzugsweise mit der elektrischen Konstantstromquelle verbunden, die der elektromagnetische Strahlung emittierenden Vorrichtung zugeordnet ist. Besonders bevorzugt ist die medizinische oder dentale Instrumententeil-Steuerung kommunikativ mit der elektrischen Konstantstromquelle verbunden. Besonders bevorzugt ist die medizinische oder dentale Instrumententeil-Steuerung dazu ausgebildet, Steuersignale an die elektrische Konstantstromquelle zu übermitteln, um die Emission von elektromagnetischer Strahlung durch die elektromagnetische Strahlung emittierende Vorrichtung zu steuern. Besonders bevorzugt sind die medizinische oder dentale Instrumententeil-Steuerung und die elektromagnetische Strahlung emittierende Vorrichtung, insbesondere die elektrische Konstantstromquelle, über elektrische Steuerleitungen miteinander kommunikativ verbunden. Besonders bevorzugt ist die elektrische Konstantstromquelle der elektromagnetische Strahlung emittierenden Vorrichtung ausgebildet, Steuersignale der medizinischen oder dentalen Instrumenten-Steuerung zu empfangen und die elektromagnetische Strahlung emittierenden Vorrichtung entsprechend den empfangenen Steuersignalen zu betreiben.

In Bezug auf jedes oben und im Folgenden beschriebene Ausführungsbeispiel und sofern nicht ausdrücklich anders beschrieben, kann der zumindest eine Sensor des medizinischen oder dentalen Instrumententeils zumindest einen der folgenden Sensoren umfassen: einen mechanischen Sensor; einen kapazitiven Sensor; einen induktiven Sensor; einen optischen oder optoelektronischen Sensor, insbesondere einen Bilderfassungssensor, eine Kamera oder einen Sensor, der eine elektromagnetische Strahlungsintensität oder eine Wellenlänge erfasst; einen magnetischen Sensor, insbesondere einen Hall-Sensor oder einen Reed-Sensor; einen akustischen oder Schall-Sensor, insbesondere ein Mikrofon oder einen Ultraschallsensor; einen thermoelektrischen Sensor; einen piezoelektrischen Sensor; einen magnetostriktiven Sensor; einen magnetoresistiven Sensor; einen elektrochemischen Sensor; einen resistiven Sensor, insbesondere einen Thermistor; einen chemischen Sensor; einen analogen Sensor, der insbesondere ein analoges Signal abgibt; einen digitalen Sensor, der insbesondere ein digitales Signal abgibt; einen virtuellen Sensor; einen MEMS- (Micro-Electro-Mechanical System) Sensor; einen berührungsempfindlichen Sensor.

Der zumindest eine Sensor kann an zumindest einer der folgenden Positionen angeordnet sein: im Inneren des medizinischen oder dentalen Instrumententeils; an der Außenseite des medizinischen oder dentalen Instrumententeils; an einer oder mehreren Komponenten des medizinischen oder dentalen Instrumententeils; an einer Außenhülse oder Griffhülse des medizinischen oder dentalen Instrumententeils; an einem Lager des medizinischen oder dentalen Instrumententeils; an einer Leiterplatte des medizinischen oder dentalen Instrumententeils. Alternativ kann der zumindest eine Sensor mit dem medizinischen oder dentalen Instrumententeil verbunden und von diesem entfernt werden, z.B. wenn der zumindest eine Sensor in einem Element enthalten ist, das mit dem medizinischen oder dentalen Instrumententeil gekoppelt werden kann.

Der zumindest eine Sensor kann so ausgebildet sein, dass er zumindest einen der folgenden Parameter erfasst: Temperatur; Geschwindigkeit; Drehmoment; Kraft; Vibration; Druck eines Fluids, insbesondere einer Flüssigkeit oder eines Gases; Feuchtigkeit; Entfernung; Konzentration einer Substanz, insbesondere in Gewebe oder einer Körperflüssigkeit; Zeit; elektrische Impedanz; elektrische Spannung; elektrische Stromstärke; Dichte eines Materials; eine Eingabe, Geste oder Angabe eines Benutzers; eine Eingabe, Geste oder Angabe einer Person, die mit dem medizinischen oder dentalen Instrumententeil behandelt wird.

Besonders bevorzugt kann der zumindest eine Sensor einen Beschleunigungsmesser (Accelerometer) oder ein Gyroskop umfassen. Die von einem Beschleunigungsmesser oder einem Gyroskop bereitgestellten Sensorsignale können insbesondere von der medizinischen oder dentalen Instrumententeil-Steuerung und/oder von der Steuer-, Regel- oder Versorgungseinheit verwendet werden, um die Position des medizinischen oder dentalen Instrumententeils zu verfolgen und/oder um das medizinische oder dentale Instrumententeil durch Gestensteuerung zu steuern und/oder um das medizinische oder dentale Instrumententeil zu navigieren.

Gemäß einer Ausführungsbeispiel ist die erste gemeinsame elektrische Leitung mit einem ersten elektrischen Kontakt an einer Stirnseite der Kupplungsvorrichtung des medizinischen oder dentalen Instrumententeils verbunden, die zweite gemeinsame elektrische Leitung ist mit einem zweiten elektrischen Kontakt an der Stirnseite der Kupplungsvorrichtung des medizinischen oder dentalen Instrumententeils verbunden und die dritte elektrische Leitung ist mit einem dritten elektrischen Kontakt an der Stirnseite der Kupplungsvorrichtung des medizinischen oder dentalen Instrumententeils verbunden. Der erste, der zweite und der dritte elektrische Kontakt können mit entsprechenden Kontakten der Versorgungsleitung zur Versorgung der elektromagnetische Strahlung emittierenden Vorrichtung und der medizinischen oder dentalen Instrumententeil-Steuerung mit elektrischer Energie und zur Übertragung des Sensorsignals und vorzugsweise der Identifikations- und/oder Betriebs- und/oder Pflegedaten verbunden werden. Der erste, der zweite und der dritte elektrische Kontakt sind an einem gemeinsamen Vorsprung angeordnet, der aus der Stirnseite der Kopplungseinrichtung herausragt.

Gemäß einem Ausführungsbeispiel umfasst eine medizinische oder dentale Behandlungsvorrichtung ein im Vorstehenden beschriebenes, medizinisches oder dentales Instrumententeil; eine gemeinsame elektrische Energiequelle zur Versorgung des medizinischen oder dentalen Instrumententeils mit elektrischer Energie, insbesondere der elektromagnetische Strahlung emittierenden Vorrichtung und der medizinischen oder dentalen Instrumententeil-Steuerung sowie des zumindest einen Sensors, wenn der Sensor Energie zum Betrieb benötigt; und eine Steuer-, Regel- oder Versorgungseinheit zum Empfang des Sensorsignals des zumindest einen Sensors und vorzugsweise zum Austausch von Identifikations- und/oder Betriebs- und/oder Pflegedaten des Instrumententeils mit der medizinischen oder dentalen Instrumententeil-Steuerung. Vorzugsweise umfasst die gemeinsame elektrische Energiequelle eine Konstantspannungsquelle.

Die Steuer-, Regel- oder Versorgungseinheit ist insbesondere so ausgebildet, dass sie zumindest einen der folgenden Schritte ausführt: Betreiben des medizinischen oder dentalen Instrumententeils auf der Grundlage des von dem zumindest einen Sensor empfangenen Sensorsignals; Speichern des von dem zumindest einen Sensor empfangenen Sensorsignals; Anzeigen eines von dem von dem zumindest einen Sensor empfangenen Sensorsignal abgeleiteten Werts oder Parameters.

Gemäß einem Ausführungsbeispiel umfasst eine medizinische oder dentale Behandlungsvorrichtung ein erstes medizinisches oder dentales Instrumententeil, das eine erste, mit elektrischer Energie betreibbare, medizinische oder dentale Instrumententeil- Steuerung, eine elektromagnetische Strahlung emittierende Vorrichtung und zumindest einen ersten Sensor umfasst, der so ausgebildet ist, dass er ein erstes Sensorsignal erzeugt; ein zweites medizinisches oder dentales Instrumententeil, das von dem ersten medizinischen oder dentalen Instrumententeil verschieden ist und eine zweite, mit elektrischer Energie betreibbare, medizinische oder dentale Instrumententeil-Steuerung und zumindest einen zweiten Sensor umfasst, der so ausgebildet ist, dass er ein zweites Sensorsignal erzeugt; und drei elektrische Leitungen, die eine erste gemeinsame elektrische Leitung, eine zweite gemeinsame elektrische Leitung und eine dritte elektrische Leitung umfassen. Das erste medizinische oder dentale Instrumententeil und das zweite medizinische oder dentale Instrumententeil sind miteinander verbindbar, um über einen gemeinsamen Versorgungsschlauch an eine gemeinsame elektrische Stromquelle und an eine gemeinsame Steuer-, Regel- oder Versorgungseinheit angeschlossen zu werden. Die erste gemeinsame elektrische Leitung und die zweite gemeinsame elektrische Leitung sind mit der ersten und der zweiten, medizinischen oder dentalen Instrumententeil-Steuerung und mit der elektromagnetischen Strahlungsemissionsvorrichtung verbunden und versorgen die elektromagnetische Strahlung emittierende Vorrichtung zum Betrieb der elektromagnetische Strahlung emittierenden Vorrichtung und die erste und zweite medizinische oder dentale Instrumententeil-Steuerung zum Betrieb der ersten und zweiten medizinischen oder dentalen Instrumententeil-Steuerung mit elektrischer Energie von der gemeinsamen elektrischen Energiequelle. Die dritte elektrische Leitung der drei elektrischen Leitungen ist mit dem zumindest einen ersten und zweiten Sensor verbunden, so dass die ersten und zweiten Sensorsignale des zumindest einen ersten und zweiten Sensors über die dritte elektrische Leitung an die gemeinsame Steuer-, Regel- oder Versorgungseinheit übertragen werden können.

Gemäß diesem Ausführungsbeispiel erfolgt die elektrische Energieversorgung und der Datentransfer zwischen dem ersten medizinischen oder dentalen Instrumententeil, dem zweiten medizinischen oder dentalen Instrumententeil und der gemeinsamen Steuer-, Regel- oder Versorgungseinheit in vorteilhafter Weise über die drei elektrischen Leitungen. Die Anzahl der elektrischen Leitungen zwischen den medizinischen oder dentalen Instrumententeilen und der gemeinsamen Steuer-, Regel- oder Versorgungseinheit wird dadurch gering gehalten, was die Herstellung erleichtert und die Herstellungskosten der medizinischen oder dentalen Behandlungsvorrichtung reduziert.

Das erste medizinische oder dentale Instrumententeil und das zweite medizinische oder dentale Instrumententeil können eines der folgenden, aber nicht dieselben Elemente umfassen: ein in der Hand haltbares medizinisches oder dentales Element, z. B. ein Griffelement, ein gerades Handstück, ein gewinkeltes oder gebogenes Handstück oder ein Winkelstück; einen Adapter; einen Aufsatz; ein Kupplungselement; ein Antriebselement, z.B. einen Druckluftmotor oder einen Elektromotor, insbesondere zum Antrieb eines Bauteils oder eines Werkzeugs, das an einem in der Hand haltbaren medizinischen oder dentalen Element anbringbar ist; einen Versorgungsschlauch, insbesondere einen Kupplungsabschnitt eines Versorgungsschlauchs, der zum Ankuppeln an ein in der Hand haltbares medizinisches oder dentales Element oder an ein Antriebselement ausgebildet ist.

Das erste medizinische oder dentale Instrumententeil entspricht den oben beschriebenen Ausführungsbeispielen der medizinischen oder dentalen Instrumententeile und kann eines oder mehrere der Elemente, Merkmale oder Funktionen umfassen, die im Zusammenhang mit diesen vorherigen Ausführungsbeispielen beschrieben wurden. Insbesondere entsprechen die erste medizinische oder dentale Instrumententeil-Steuerung, die elektromagnetische Strahlung emittierende Vorrichtung und der zumindest eine erste Sensor des ersten medizinischen oder dentalen Instrumententeils den jeweiligen Ausführungsbeispielen der medizinischen oder dentalen Instrumententeil-Steuerung, des zumindest einen Sensors und der elektromagnetische Strahlung emittierenden Vorrichtung, die oben beschrieben wurden, und können daher eines oder mehrere der Elemente, Merkmale oder Funktionen umfassen, die im Zusammenhang mit diesen vorherigen Ausführungsbeispielen beschrieben wurden.

Dementsprechend korrespondieren die zweite medizinische oder dentale Instrumententeil-Steuerung und der zumindest eine zweite Sensor des zweiten medizinischen oder dentalen Instrumententeils den oben beschriebenen Ausführungsbeispielen der medizinischen oder dentalen Instrumententeil-Steuerungen und des zumindest einen Sensors und können somit eines oder mehrere der Elemente, Merkmale oder Funktionen umfassen, die im Zusammenhang mit diesen vorherigen Ausführungsbeispielen beschrieben wurden.

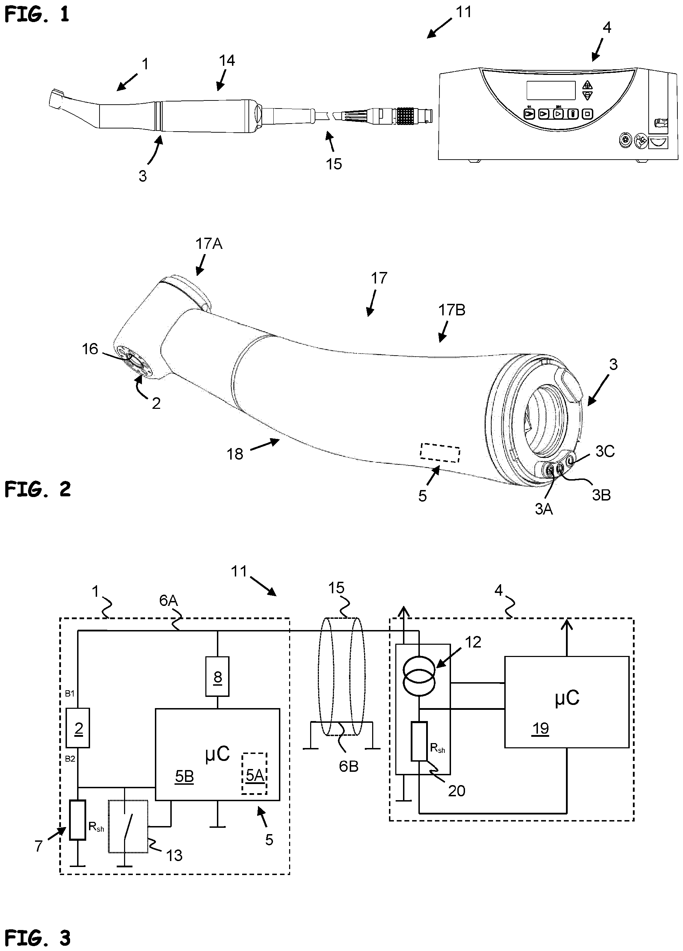

Die Erfindung wird nachfolgend anhand bevorzugter Ausführungsbeispiele und Bezug nehmend auf die beigefügten Zeichnungen erläutert. Die in den Figuren 3 bis 5 dargestellten Ausführungsbeispiele sind nicht Teil der beanspruchten Erfindung. Die in den Figuren 1, 2 und 6 bis 11 dargestellten Ausführungsbeispiele sind Teil der beanspruchten Erfindung.
Figur 1 zeigt eine medizinische oder dentale Behandlungsvorrichtung, umfassend: ein medizinisches oder dentales Instrumententeil mit einer Beleuchtungsvorrichtung, einer Speichervorrichtung und zumindest einer gemeinsamen elektrischen Leitung für die Versorgung der Beleuchtungsvorrichtung und der Speichervorrichtung mit elektrischer Energie, eine Antriebsvorrichtung, einen Versorgungsschlauch und eine Steuer-, Regel- oder Versorgungseinheit mit einer elektrischen Energiequelle zur Versorgung der Beleuchtungsvorrichtung und der Speichervorrichtung mit elektrischer Energie.
Figur 2 zeigt das medizinische oder dentale Instrumententeil der Figur 1.
Figur 3 zeigt eine schematische Darstellung eines ersten, nicht erfindungsgemäßen Ausführungsbeispiels eines elektrischen Schalt- oder Stromkreises mit zumindest einer gemeinsamen elektrischen Leitung für die Versorgung einer Beleuchtungsvorrichtung und einer Speichervorrichtung.

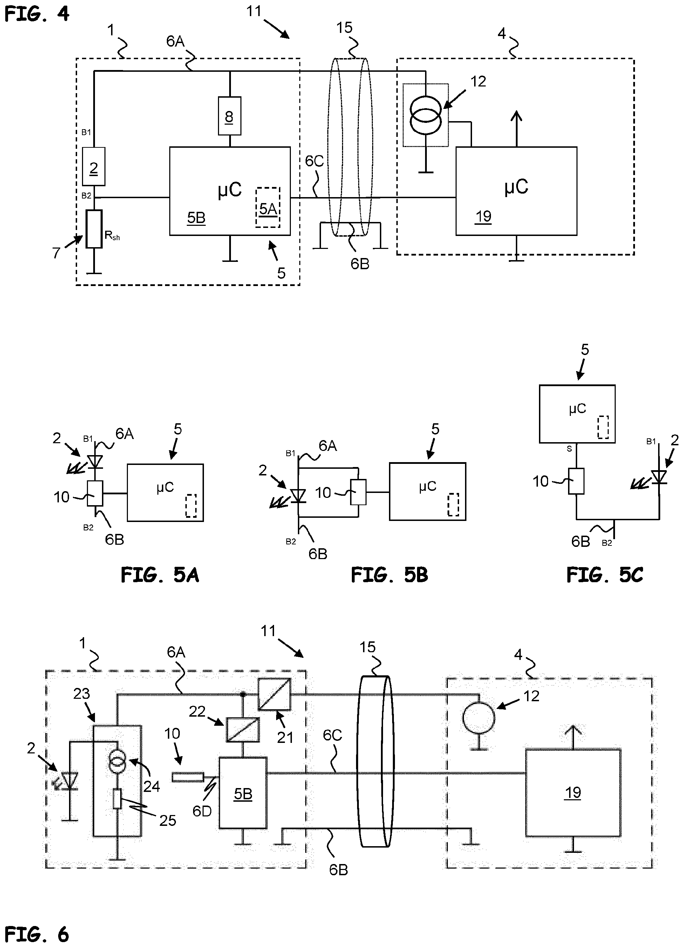

Figur 4 zeigt eine schematische Darstellung eines zweiten Ausführungsbeispiels eines elektrischen Schalt- oder Stromkreises mit zumindest einer gemeinsamen elektrischen Leitung für die Versorgung einer Beleuchtungsvorrichtung und einer Speichervorrichtung.
Figur 5A-5C zeigen drei unterschiedliche Ausführungsformen einer Beleuchtungsvorrichtung und eines Sensors für einen elektrischen Schalt- oder Stromkreis der Figuren 4 oder 3.
Figur 6 zeigt eine schematische Darstellung eines Ausführungsbeispiels einer elektrischen Schaltung, die ein medizinisches oder dentales Instrumententeil mit einer Steuer-, Regel- oder Versorgungseinheit verbindet, wobei die elektrische Schaltung zwei elektrische Leitungen zur Versorgung einer Strahlung emittierenden Vorrichtung, einer Steuerung und optional eines Sensors des medizinischen oder dentalen Instrumententeils mit elektrischer Energie und eine weitere elektrische Leitung zur Verbindung des Sensors mit der Steuer-, Regel- oder Versorgungseinheit zur Übertragung eines Sensorsignals aufweist.

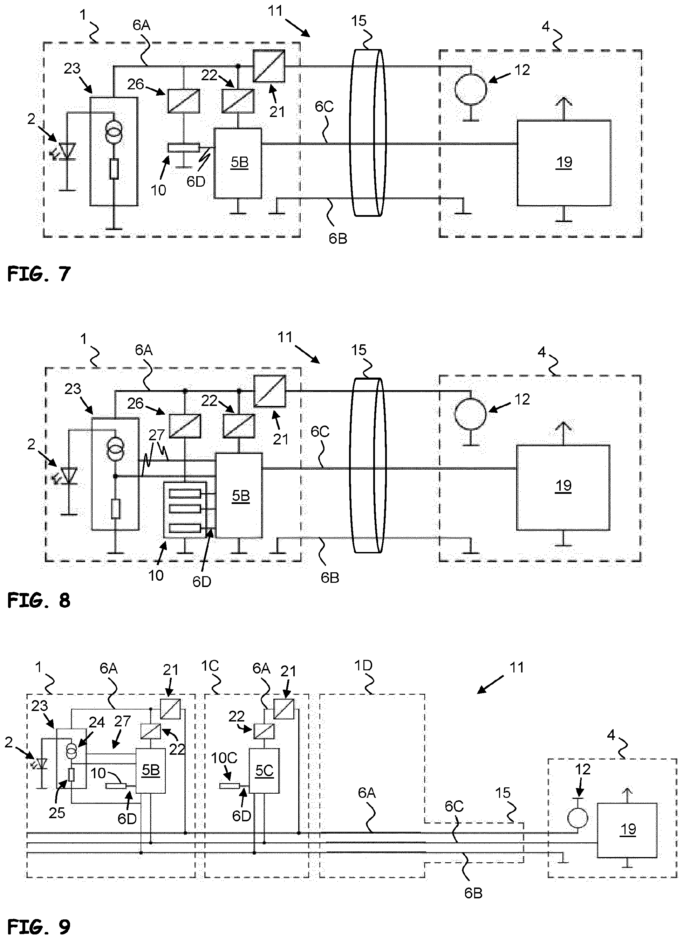

Figur 7 zeigt eine schematische Darstellung eines Ausführungsbeispiels einer elektrischen Schaltung, die ein medizinisches oder dentales Instrumententeil mit einer Steuer-, Regel- oder Versorgungseinheit verbindet, wobei die elektrische Schaltung zwei elektrische Leitungen zur Versorgung einer Strahlung emittierenden Vorrichtung, einer Steuerung und eines Sensors des medizinischen oder dentalen Instrumententeils mit elektrischer Energie und eine weitere elektrische Leitung zur Verbindung des Sensors mit der Steuer-, Regel- oder Versorgungseinheit zur Übertragung eines Sensorsignals aufweist.
Figur 8 zeigt ein schematisches Diagramm eines Ausführungsbeispiels einer elektrischen Schaltung, die ein medizinisches oder dentales Instrumententeil mit einer Steuer-, Regel- oder Versorgungseinheit verbindet, wobei die elektrische Schaltung zwei elektrische Leitungen zur Versorgung einer Strahlung emittierenden Vorrichtung, einer Steuerung und mehreren Sensoren des medizinischen oder dentalen Instrumententeils mit elektrischer Energie und eine weitere elektrische Leitung zur Verbindung der mehreren Sensoren mit der Steuer-, Regel- oder Versorgungseinheit zur Übertragung eines Sensorsignals aufweist.
Figur 9 zeigt eine schematische Darstellung eines Ausführungsbeispiels einer elektrischen Schaltung, die ein medizinisches oder dentales Behandlungsgerät mit einer Steuer-, Regel- oder Versorgungseinheit verbindet, wobei die elektrische Schaltung zwei elektrische Leitungen zur Versorgung einer Strahlung emittierenden Vorrichtung, mehrerer Steuerungen und mehrerer Sensoren des medizinischen oder dentalen Behandlungsgeräts mit elektrischer Energie und eine weitere elektrische Leitung zur Verbindung der mehreren Sensoren mit der Steuer-, Regel- oder Versorgungseinheit zur Übertragung eines Sensorsignals aufweist.

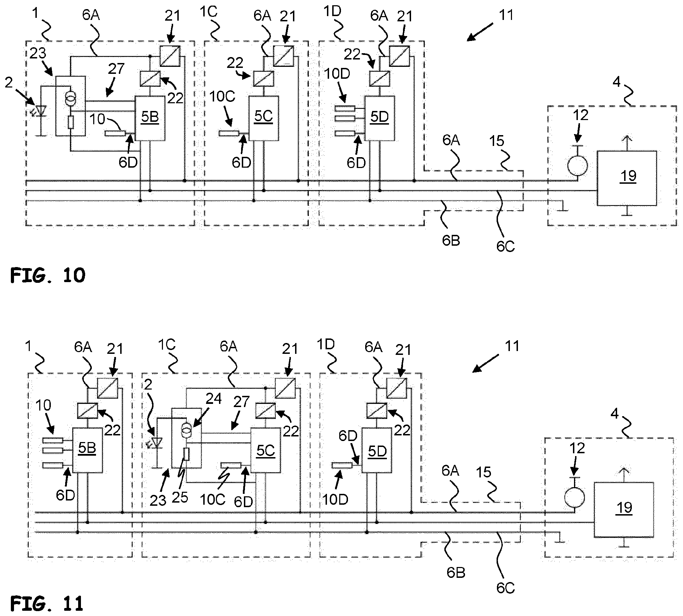

Figur 10 zeigt ein schematisches Diagramm eines anderen Ausführungsbeispiels einer elektrischen Schaltung, die eine medizinische oder dentale Behandlungsvorrichtung mit einer Steuer-, Regel- oder Versorgungseinheit verbindet, wobei die elektrische Schaltung zwei elektrische Leitungen zur Versorgung einer Strahlung emittierenden Vorrichtung, mehrerer Steuerungen und mehrerer Sensoren der medizinischen oder dentalen Behandlungsvorrichtung mit elektrischer Energie und eine weitere elektrische Leitung zur Verbindung der mehreren Sensoren mit der Steuer-, Regel- oder Versorgungseinheit zur Übertragung eines Sensorsignals aufweist.
Figur 11 zeigt ein schematisches Diagramm eines anderen Ausführungsbeispiels einer elektrischen Schaltung, die eine medizinische oder dentale Behandlungsvorrichtung mit einer Steuer-, Regel- oder Versorgungseinheit verbindet, wobei die elektrische Schaltung zwei elektrische Leitungen zur Versorgung einer Strahlung emittierenden Vorrichtung, mehreren Steuerungen und mehreren Sensoren der medizinischen oder dentalen Behandlungsvorrichtung mit elektrischer Energie und eine weitere elektrische Leitung zur Verbindung der Mehrzahl von Sensoren mit der Steuer-, Regel- oder Versorgungseinheit zur Übertragung eines Sensorsignals aufweist.

Die Figur 1 zeigt eine medizinische oder dentale Behandlungsvorrichtung 11, die ein medizinisches oder dentales Instrumententeil 1, eine Antriebseinheit 14, eine Steuer-, Regel- oder Versorgungseinheit 4 und einen das Instrumententeil 1 mit der Steuer-, Regel- oder Versorgungseinheit 4 verbindenden Versorgungsschlauch 15 umfasst. Das Instrumententeil 1 ist über eine Kupplungsvorrichtung 3 lösbar mit der Steuer-, Regel- oder Versorgungseinheit 4 verbunden.

Das in der Figur 2 vergrößert dargestellte Instrumententeil 1 ist durch ein gebogenes Handstück oder Winkelstück 17 gebildet. Das Handstück 17 umfasst einen Handstückkopf 17A, in dem eine Werkzeughalterung zur, insbesondere lösbaren, Befestigung eines Werkzeugs angeordnet ist, sowie einen Griffteil 17B zum Halten der Handstücks 17 mit einer Hand. Am freien oder proximalen Ende des Griffteils 17B ist die Kupplungsvorrichtung 3 bzw. zumindest ein Teil davon angeordnet.

Die Antriebseinheit 14 umfasst zum Beispiel einen E-Motor oder einen Luftmotor, die als separates, von dem Handstück 17 lösbares Bauteil (wie in Figur 1 dargestellt) oder in das Handstück 17 integriert ausgebildet sind. Die Antriebseinheit 14 kann jedoch auch ein in das Handstück 17, insbesondere im Handstückkopf 17A, angeordnetes, mit einem Treibgas angetriebenes Laufrad umfassen. Das Handstück 17 kann somit motorbetrieben sein und zumindest eine Antriebswelle zum Übertragen einer Antriebsbewegung auf das Werkzeug aufweisen oder treibgasbetrieben mit einem Laufrad ausgebildet sein. In beiden Fällen umfasst das Handstück 17 zumindest ein in Drehung versetzbares Antriebselement zum Antrieb eines mit dem Handstück verbindbaren Werkzeugs.

Das Handstück 17 umfasst des Weiteren eine Beleuchtungsvorrichtung 2, die vorzugsweise am Handstückkopf 17A oder daran anschließend angeordnet ist. Die in der Figur 2 dargestellte Beleuchtungsvorrichtung 2 umfasst insbesondere mehrere optische Halbleiterelemente (LEDs), die ringförmig um eine Werkzeugaufnahmeöffnung 16 des Handstückkopfs 17A angeordnet sind.

Bevorzugt sind des Weiteren um die Werkzeugaufnahmeöffnung 16, vorzugsweise alternierend mit den optischen Halbleiterelementen, Öffnungen zur Abgabe eines Mediums, zum Beispiel von Wasser und / oder Luft, angeordnet. Die Öffnungen zur Abgabe eines Mediums sind über zumindest eine Medienleitung in dem Handstück 17 mit der Steuer-, Regel- oder Versorgungseinheit 4 zum Zuführen zumindest eines Mediums verbunden.

In dem Instrumententeil 1 oder Handstück 17 ist des Weiteren eine mit elektrischer Energie betreibbare Speichervorrichtung 5 zur Speicherung von Identifikationsdaten und / oder Betriebsdaten und / oder Pflegedaten des Instrumententeils 1 oder Handstücks 17 angeordnet. Die Speichervorrichtung 5 kann im Handstückkopf 17A oder im Griffteil 17B angeordnet sein oder es kann jeweils ein Teil der Speichervorrichtung 5 im Handstückkopf 17A und im Griffteil 17B vorgesehen sein. Innerhalb des Griffteils 17B ist die Speichervorrichtung 5 oder ein Teil davon zum Beispiel an dessen proximalen oder freien Ende, insbesondere in oder an der Kupplungsvorrichtung 3, in einem unmittelbar an den Handstückkopf 17A anschließenden Abschnitt des Griffteils 17B oder in einer Biegung 18 des Griffteils 17B angeordnet.

Die Speichervorrichtung 5 umfasst insbesondere ein Speicherelement 5A und einen Mikrocontroller 5B, insbesondere zum Betreiben des Speicherelements 5A (siehe Figuren 3 und 4).

Wie insbesondere aus den Figuren 3 und 4 zu erkennen ist, umfasst das Instrumententeil 1 oder das Handstück 17 des Weiteren zumindest eine (gemeinsame) elektrische Leitung 6A, 6B, die für die Versorgung der an dem Handstück 17 vorgesehenen Beleuchtungsvorrichtung 2 mit elektrischer Energie zum Betrieb der Beleuchtungsvorrichtung 2 und für die Versorgung der Speichervorrichtung 5 mit elektrischer Energie zum Betrieb der Speichervorrichtung 5 vorgesehen ist. Die zumindest eine (gemeinsame) elektrische Leitung 6A, 6B verbindet die Beleuchtungsvorrichtung 2 und die Speichervorrichtung 5 mit einer in der Steuer-, Regel- oder Versorgungseinheit 4 angeordneten elektrischen Energiequelle 12, insbesondere eine Konstantstromquelle, die ausgebildet ist, die Beleuchtungsvorrichtung 2 und die Speichervorrichtung 5 mit elektrischer Energie zu versorgen. Wie aus den Figuren 3 und 4 auch zu erkennen ist, erstreckt sich die zumindest eine (gemeinsame) elektrische Leitung 6A, 6B durch den Versorgungsschlauch 15 bis zu oder in die Steuer-, Regel- oder Versorgungseinheit 4.

Die zumindest eine (gemeinsame) elektrische Leitung 6A, 6B verbindet somit elektrisch die Beleuchtungsvorrichtung 2 und die Speichervorrichtung 5 mit der Steuer-, Regel- oder Versorgungseinheit 4, insbesondere deren elektrischer Energiequelle 12 und Mikrocontroller 19, wodurch ein elektrischer Schalt-, Steuer-, Regel- oder Versorgungskreis gebildet ist. Vorzugsweise umfasst der Schalt-, Steuer-, Regel- oder Versorgungskreis auch noch zumindest einen Sensor 10, wie im Folgenden noch im Detail beschrieben ist.

Die elektrische Energiequelle 12 in Form einer Konstantstromquelle versorgt das Instrumententeil 1 oder das Handstück 17 mit elektrischer Energie mit konstanter elektrischer Stromstärke. Dies bildet für die zumindest einen optischen Halbleiter umfassende Beleuchtungsvorrichtung 2 eine optimale Energieversorgung. Die Speichervorrichtung 5, insbesondere der Mikrocontroller 5B, benötigt jedoch eine elektrische Versorgung mit einer konstanten elektrischen Spannung. Dazu ist eine der Speichervorrichtung 5, insbesondere dem Mikrocontroller 5B, zugeordnete Vorrichtung 8 zur Spannungsaufbereitung vorgesehen, die ausgebildet ist, die von der elektrischen Energiequelle 12 (über die elektrische Leitung 6A, 6B) empfangene elektrischer Energie mit konstanter elektrischer Stromstärke in elektrische Energie mit konstanter elektrischer Spannung zu wandeln und der Speichervorrichtung 5 zuzuführen. Die Vorrichtung 8 zur Spannungsaufbereitung ist insbesondere in dem Instrumententeil 1 oder das Handstück 17 angeordnet.

Gemäß der Figur 3 sind zwei (gemeinsame) elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zusätzlich sind die zwei elektrischen Leitungen 6A, 6B zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 vorgesehen. In anderen Worten sind somit für die Versorgung der Beleuchtungsvorrichtung 2 und der Speichervorrichtung 5 mit elektrischer Energie und für die Datenübertragung nicht mehr als die zwei (gemeinsamen) elektrischen Leitungen 6A, 6B vorgesehen. Entsprechend ist die Steuer-, Regel- oder Versorgungseinheit 4 zum Austausch von Identifikationsdaten und / oder Betriebsdaten und / oder Pflegedaten des Instrumententeils 1 mit der Speichervorrichtung 5 ausgebildet.

Zur Übertragung von Identifikationsdaten und / oder Betriebsdaten und / oder Pflegedaten von der Speichervorrichtung 5 und zu der Steuer-, Regel- oder Versorgungseinheit 4, d.h. zum Auslesen von Daten aus der Speichervorrichtung 5, ist ein der Speichervorrichtung 5 zugeordnetes elektrisches Schaltelement 13 vorgesehen. Wie im Vorstehenden bereits beschrieben ist das Schaltelement 13 ausgebildet, Änderungen eines elektrischen Stromparameters zu bewirken, insbesondere der elektrischen Spannung oder der elektrischen Last, insbesondere durch das Kurzschließen eines Shunt-Widerstands 7. Die Änderungen des elektrischen Stromparameters definieren (digital) die übertragenen Daten. Die Steuer-, Regel- oder Versorgungseinheit 4, insbesondere deren Mikrocontroller 19, ist ausgebildet, die Änderungen des elektrischen Stromparameters zu detektieren oder auszugleichen und daraus die (digital) übertragenen Identifikationsdaten und / oder Betriebsdaten und / oder Pflegedaten zu erkennen oder auszulesen.

Im Instrumententeil 1 oder Handstück 17 ist des Weiteren einen Shunt-Widerstand 7 vorgesehen, der ausgebildet ist, Änderungen der elektrischen Stromstärke zur Übertragung von Identifikationsdaten und / oder Betriebsdaten und / oder Pflegedaten zwischen der Speichervorrichtung 5 und der mit dem Instrumententeil 1 verbindbaren Steuer-, Regel- oder Versorgungseinheit 4 aufzubereiten. Wie im Vorstehenden bereits beschrieben ist der Shunt-Widerstand 7 zum Abspeichern von Daten in der Speichervorrichtung 5 vorgesehen, insbesondere zur Übertragen von Daten von der Steuer-, Regel- oder Versorgungseinheit 4 zu der Speichervorrichtung 5.

Ein weiterer Shunt-Widerstand 20 ist der Steuer-, Regel- oder Versorgungseinheit 4, insbesondere dem Mikrocontroller 19 der Steuer-, Regel- oder Versorgungseinheit 4, zugeordnet.

Gemäß der Figur 4 sind zwei (gemeinsame) elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 ist eine separate Leitung 6C oder Datenleitung vorgesehen. In anderen Worten sind somit für die Versorgung der Beleuchtungsvorrichtung 2 und der Speichervorrichtung 5 mit elektrischer Energie und für die Datenübertragung drei Leitungen vorgesehen, nämlich die zwei (gemeinsamen) elektrischen Leitungen 6A, 6B für die Versorgung mit elektrischer Energie und die separate Leitung 6C für die Übertragung der Daten, so wie dies im Vorstehenden beschrieben ist.

Die separate Datenleitung 6C kann als elektrische Leitung zum Übertragen elektrischer (Daten-)Signale oder als optische Leitung, zum Beispiel als Glasfaser, zum Übertragen optischer (Daten-)Signale ausgebildet sein. Die separate Leitung 6C erstreckt sich durch das Instrumententeil 1 oder Handstück 17 und den Versorgungsschlauch 15 bis zu oder in die Steuer-, Regel- oder Versorgungseinheit 4.

An der Kupplungsvorrichtung 3 des Instrumententeils 1 oder Handstücks 17, insbesondere an der Stirnfläche der Kupplungsvorrichtung 3, sind elektrische Kontakte 3A, 3B vorgesehen, die mit der zumindest einen gemeinsamen elektrischen Leitung 6A, 6B verbunden sind (siehe Figur 2). Diese lösbaren elektrischen Kontakte 3A, 3B verbinden die Beleuchtungsvorrichtung 2 und die Speichervorrichtung 5 elektrisch mit der Steuer-, Regel- oder Versorgungseinheit 4, insbesondere mit deren Energiequelle 12 und Mikrocontroller 19. Wenn nur zwei gemeinsame elektrische Leitungen 6A, 6B zur Übertragung der elektrischen Energie und von Daten vorgesehen sind, so wie dies im Vorstehenden beschrieben ist, dann sind in entsprechender Weise auch nur diese zwei elektrische Kontakte 3A, 3B an der Kupplungsvorrichtung 3 vorgesehen. Wenn zumindest eine separate optische oder elektrische Leitung 6C zum Übertragen von Identifikationsdaten und / oder Betriebsdaten und / oder Pflegedaten vorhanden ist, dann ist in entsprechender Weise ein weiterer optischer oder elektrischer Kontakt 3C vorgesehen, der mit dieser zumindest einen separaten Leitung 6C für die Datenübertragung verbunden ist.

Vorzugsweise ist der zumindest eine elektrische Kontakt oder sind die elektrischen Kontakte lösbar mit der Steuer-, Regel- oder Versorgungseinheit 4 verbunden. Der zumindest eine elektrische Kontakt ist zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

In dem medizinischen oder dentalen Instrumententeil 1 oder Handstück 17 ist vorzugsweise zumindest einen Sensor 10 vorgesehen, der ausgebildet ist, einen Betriebszustand des Instrumententeils 1 oder Handstücks 17 zu detektieren und ein Sensorsignal zu erzeugen. Der Sensor 10 umfasst zum Beispiel einen Temperatursensor oder einen Drehzahlsensor zum Messen der Drehzahl eines Antriebselements der Instrumententeils 1 / Handstücks 17. In den Figuren 5A - 5C sind unterschiedliche Anordnungen des Sensors 10 dargestellt.

Der zumindest eine Sensor 10 ist mit der zumindest einen elektrischen Leitung 6A, 6B elektrisch verbunden, so dass das Sensorsignal des Sensors 10 und / oder elektrische Energie zum Betreiben des Sensors 10 über die zumindest eine elektrische Leitung 6A, 6B übertragbar ist/sind.

Der zumindest eine Sensor 10 ist vorzugsweise auch mit der Speichervorrichtung 5, insbesondere dem Mikrocontroller 5B, elektrisch verbunden, so dass insbesondere ein Sensorsignal des Sensors 10 an die Speichervorrichtung 5 weiterleitbar ist. Die Speichervorrichtung 5, insbesondere der Mikrocontroller 5B, sind wahlweise ausgebildet (1) das (analoge) Sensorsignal des Sensors 10 weiterzuleiten, vorzugsweise an die Steuer-, Regel- oder Versorgungseinheit 4, insbesondere an deren Mikrocontroller 19, oder (2) das analoge Sensorsignal in ein digitales Signal zu wandeln und an die Steuer-, Regel- oder Versorgungseinheit 4, insbesondere an deren Mikrocontroller 19, zu leiten oder (3) das (analoge) Sensorsignal des Sensors 10 zu empfangen und zu verarbeiten und das Instrumententeil 1 / Handstück 17 oder ein Bauteil davon auf Basis des Sensorsignals zu betreiben, zu regeln oder zu steuern. Das Weiterleiten des Sensorsignals an die Steuer-, Regel- oder Versorgungseinheit 4 gemäß (1) oder (2) erfolgt vorzugsweise über zumindest eine der Leitungen 6A, 6B, 6C.

Bei der Ausführungsform der Figur 5A ist der Sensor 10, vorzugsweise ein Sensor zum Messen der Drehzahl, elektrisch in Serie mit der Beleuchtungsvorrichtung 2 angeordnet. Bei der Ausführungsform der Figur 5B ist der Sensor 10, vorzugsweise ein Sensor zum Messen der Temperatur, elektrisch parallel mit der Beleuchtungsvorrichtung 2 angeordnet. In beiden Ausführungsformen weisen der Sensor 10 und die Beleuchtungsvorrichtung 2 eine gemeinsame elektrische Energiequelle 12 auf und sie sind mit den elektrischen Leitung 6A, 6B elektrisch verbunden.

Bei der Ausführungsform der Figur 5C sind der Sensor 10, vorzugsweise ein Sensor zum Messen der Temperatur, und die Beleuchtungsvorrichtung 2 mit unterschiedlichen elektrischen Energiequellen zur Versorgung mit elektrischer Energie verbunden.

Die Erfindung ist nicht auf die dargestellten und beschriebenen Ausführungsbeispiele beschränkt. Des Weiteren sind alle Merkmale aller beschriebenen und dargestellten Ausführungsbeispiele miteinander kombinierbar.

Als nächstes werden die Figuren 6 - 8 erörtert, die verschiedene Ausführungsformen von medizinischen oder dentalen Behandlungsvorrichtungen 11 gemäß der beanspruchten Erfindung zeigen. Die in den Figuren 3 und 4 gezeigten Elemente sind auch in den Figuren 6 bis 8 dargestellt und tragen dieselben Ziffern: medizinisches oder dentales Instrumententeil 1; Vorrichtung zur Emission von Strahlung oder Beleuchtungsvorrichtung 2; Steuer-, Regel- oder Versorgungseinheit 4; Steuerung oder Mikrocontroller 5B für das medizinische oder dentale Instrumententeil; drei elektrische Leitungen 6A, 6B, 6C; gemeinsame elektrische Stromquelle 12; Versorgungsschlauch 15, der das Instrumententeil 1 mit der Steuer-, Regel- oder Versorgungseinheit 4 verbindet und elektrische Leitungen 6A, 6B, 6C hält; und Mikrocontroller 19 der Steuer-, Regel- oder Versorgungseinheit 4. Alle Merkmale, Funktionen oder Eigenschaften, die oben im Zusammenhang mit diesen Elementen beschrieben wurden, können auch für die in den Figuren 6 bis 8 dargestellten Elemente gelten.

Bei dem medizinischen oder dentalen Instrumententeil 1 kann es sich vorzugsweise um ein medizinisches oder dentales Handstück 17, eine medizinische oder dentale Antriebseinheit oder einen Elektromotor 14, einen medizinischen oder dentalen Adapter, einen medizinischen oder dentalen Aufsatz oder eine medizinische oder dentale Kupplungsvorrichtung, insbesondere als Teil eines Versorgungsschlauchs 15, handeln.

Die Figuren 6 - 8 zeigen zusätzlich zumindest einen Sensor 10, der dazu ausgebildet ist, ein Sensorsignal zu erzeugen. Der zumindest eine Sensor 10 ist in oder an dem medizinischen oder dentalen Instrumententeil 1 angeordnet. Alternativ kann der zumindest eine Sensor 10 mit dem medizinischen oder dentalen Instrumententeil 1 verbunden und von diesem entfernt werden, beispielsweise wenn der zumindest eine Sensor 10 in einem Element enthalten ist, das mit dem medizinischen oder dentalen Instrumententeil 1 gekoppelt werden kann. Der zumindest eine Sensor 10 kann jeden oben beschriebenen Sensor umfassen.

Die drei in den Figuren 6 - 8 gezeigten elektrischen Leitungen 6A, 6B, 6C umfassen eine erste gemeinsame elektrische Leitung 6A und eine zweite gemeinsame elektrische Leitung 6B, die mit der medizinischen oder dentalen Instrumententeil-Steuerung 5B und der elektromagnetische Strahlung emittierenden Vorrichtung 2 verbunden sind und so ausgebildet sind, dass sie der elektromagnetische Strahlung emittierenden Vorrichtung 2 zum Betrieb der elektromagnetische Strahlung emittierenden Vorrichtung 2 und der medizinischen oder dentalen Instrumententeil-Steuerung 5B zum Betrieb der medizinischen oder dentalen Instrumententeil-Steuerung 5B elektrische Energie von der gemeinsamen elektrischen Energiequelle 12 zuführen. Benötigt der zumindest eine Sensor 10 zum Betrieb eine Stromversorgung, so wird er ebenfalls von der elektrischen Energiequelle 12 über die gemeinsamen elektrischen Leitungen 6A, 6B mit elektrischer Energie versorgt. Darüber hinaus dient eine der elektrischen Leitungen 6A, 6B, gemäß den Figuren 6 - 8 die elektrische Leitung 6B, als Masseleitung.

Die dritte elektrische Leitung 6C der drei elektrischen Leitungen verbindet die medizinische oder dentale Instrumententeil-Steuerung 5B mit der Steuer-, Regel- oder Versorgungseinheit 4, insbesondere mit dem Mikrocontroller 19. Die medizinische oder dentale Instrumententeil-Steuerung 5B kann ein Speicherelement 5A (siehe Figuren 3, 4) umfassen, das dazu ausgebildet ist, zumindest eines von Identifikations-, Betriebs- oder Pflegedaten des medizinischen oder dentalen Instrumententeils 1 und/oder eines anderen Instrumententeils, das mit dem medizinischen oder dentalen Instrumententeil 1 gekoppelt ist oder gekoppelt werden kann, zu speichern. Die dritte elektrische Leitung 6C ist zur Datenübertragung, insbesondere der Identifikationsdaten, Betriebsdaten oder Pflegedaten, zwischen dem medizinischen oder dentalen Instrumententeil-Steuerung 5B und der Steuer-, Regel- oder Versorgungseinheit 4, insbesondere dem Mikrocontroller 19, ausgebildet.

Über die dritte elektrische Leitung 6C ist der zumindest eine Sensor 10 auch mit der Steuer-, Regel- oder Versorgungseinheit 4, insbesondere mit dem Mikrocontroller 19, verbunden, so dass ein Sensorsignal des zumindest einen Sensors 10 oder auf einem Sensorsignal basierende Daten über die dritte elektrische Leitung 6C an die Steuer-, Regel- oder Versorgungseinheit 4 übertragen werden können. Wie in den Figuren 6 - 8 zu erkennen ist, wird das Sensorsignal von dem zumindest einen Sensor 10 über eine Daten- oder Signalleitung 6D an die medizinische oder dentale Instrumententeil-Steuerung 5B übertragen, von wo aus es an die Steuer-, Regel- oder Versorgungseinheit 4 weitergeleitet wird. Wie oben beschrieben, kann die Instrumententeil-Steuerung 5B entweder so ausgebildet sein, dass sie das Sensorsignal nur überträgt oder das Sensorsignal vor der Übertragung an die Steuer-, Regel- oder Versorgungseinheit 4 speichert und/oder verarbeitet.

Die elektrischen Leitungen 6A, 6B, 6C, vorzugsweise auch die Leitung 6D und/oder weitere elektrische Leitungen, können Teil eines Bussystems, insbesondere eines CAN-, Flex Ray-, Lin-, SPI- oder I²C-Bussystems, von medizinischen oder dentalen Behandlungsgeräten 11 sein.

Die gemeinsame elektrische Energiequelle 12 ist in der Steuer-, Regel- oder Versorgungseinheit 4 angeordnet und/oder bildet einen Teil der Steuer-, Regel- oder Versorgungseinheit 4. Vorzugsweise besteht die gemeinsame elektrische Energiequelle 12 aus einer Konstantspannungsquelle oder wird durch eine solche gebildet. Dementsprechend versorgt die elektrische Energiequelle 12 das medizinische oder dentale Instrumententeil 1, insbesondere die elektromagnetische Strahlungsemissionsvorrichtung 2 und die medizinische oder dentale Instrumententeil-Steuerung 5B sowie optional den zumindest einen Sensor 10 mit elektrischer Energie in Form von konstanter Spannung oder Gleichstrom.

Die Figuren 6 - 8 zeigen auch eine erste Vorrichtung zur Aufbereitung elektrischer Energie 21. Die erste Vorrichtung zur Aufbereitung elektrischer Energie 21 umfasst einen DC/DC-Wandler.

Die erste Vorrichtung zur Aufbereitung elektrischer Energie 21 ist so im medizinischen oder dentalen Instrumententeil 1 angeordnet, dass sie die von der elektrischen Energiequelle 12 gelieferte elektrische Energie für mehrere oder alle mit elektrischer Energie versorgten Elemente des medizinischen oder dentalen Instrumententeils 1 aufbereitet. Dementsprechend bereitet in den Figuren 6 - 8 die erste Vorrichtung zur Aufbereitung elektrischer Energie 21 die elektrische Energie zumindest für die medizinische oder dentale Instrumententeil-Steuerung 5B und die elektromagnetische Strahlung emittierende Vorrichtung 2 auf, und optional für den zumindest einen Sensor 10, wenn dieser für seinen Betrieb eine Energieversorgung benötigt.

Vorzugsweise liefert die elektrische Energiequelle 12 elektrische Energie mit konstanter Spannung oder Gleichstrom an die Vorrichtung zur Aufbereitung elektrischer Energie 21, die einen DC/DC-Wandler umfasst. Der DC/DC-Wandler ist so ausgebildet, dass er das Spannungsniveau der elektrischen Leistung der elektrischen Stromquelle 12 in ein anderes Spannungsniveau umwandelt. Die Vorrichtung zur Aufbereitung elektrischer Energie 21 kann zusätzlich einen Filter umfassen, um Übertragungsstörungen der elektrischen Energie zu kompensieren, die von der gemeinsamen elektrischen Energiequelle 12 über die erste und zweite gemeinsame elektrische Leitung 6A, 6B geliefert wird. Elektrischer Strom, der dieses unterschiedliche Spannungsniveau aufweist und/oder gefiltert ist, wird dann mehreren oder allen Elementen des medizinischen oder dentalen Instrumententeils 1 zugeführt, die mit elektrischem Strom versorgt werden.

Die Figuren 6 - 8 zeigen zusätzlich eine zweite Vorrichtung zur Aufbereitung elektrischer Energie 22, die nur der medizinischen oder dentalen Instrumententeil- Steuerung 5B zugeordnet ist und/oder so ausgebildet ist, dass sie elektrische Energie aufbereitet, die nur der medizinischen oder dentalen Instrumententeil-Steuerung 5B zugeführt wird. Die zweite Vorrichtung zur Aufbereitung elektrischer Energie 22 kann einen elektrischen Leistungswandler umfassen. Je nach der elektrischen Leistung, die dem der medizinischen oder dentalen Instrumententeil-Steuerung 5B zugeführt wird, kann der elektrische Leistungswandler einen DC/DC-Wandler oder einen AC/DC-Wandler oder eine elektronische Schaltung zur Umwandlung der elektrischen Leistung umfassen.

Vorzugsweise handelt es sich bei der elektrischen Energie, die der medizinischen oder dentalen Instrumententeil-Steuerung 5B zugeführt wird, insbesondere von der ersten Vorrichtung zur Aufbereitung elektrischer Energie 21, um Gleichstrom. Dementsprechend umfasst die zweite Vorrichtung zur Aufbereitung elektrischer Energie 22 einen DC/DC-Wandler oder ist ein DC/DC-Wandler, der so ausgebildet ist, dass er das Spannungsniveau der zugeführten elektrischen Energie in ein anderes Spannungsniveau umwandelt, das dem von der medizinischen oder dentalen Instrumententeil-Steuerung 5B benötigten Spannungsniveau entspricht. Elektrische Energie mit diesem unterschiedlichen Spannungsniveau wird dann der medizinischen oder dentalen Instrumententeil-Steuerung 5B zugeführt.

Die strahlungsemittierende Vorrichtung 2 umfasst vorzugsweise zumindest ein optisches Halbleiterelement oder eine Leuchtdiode (LED). LEDs benötigen zum optimalen Betrieb eine elektrische Energieversorgung mit konstanter Stromstärke. Daher umfassen die medizinischen oder dentalen Instrumententeile 1 der Figuren 6 - 8 eine Konstantstromquelle 23, die so ausgebildet ist, dass sie die Strahlung emittierende Vorrichtung 2 mit einer konstanten elektrischen Stromstärke versorgt. Die Konstantstromquelle 23 ist nur der Strahlung emittierenden Vorrichtung 22 zugeordnet und/oder versorgt nur die Strahlung emittierende Vorrichtung 2 mit elektrischer Energie.

Die elektrische Energie für die Strahlungsquelle 2, die insbesondere von der ersten Vorrichtung zur Aufbereitung elektrischer Energie 21 geliefert wird, ist vorzugsweise Gleichstrom. Die Konstantstromquelle 23 kann auch zum Anpassen der von der Strahlungsemissionsvorrichtung 2 benötigte Stromstärke ausgebildet sein. Vorzugsweise umfasst die Konstantstromquelle 23 eine Elektronik oder eine integrierte Schaltung 24, die so ausgebildet ist, dass sie die Stromstärke für die Strahlungsemissionsvorrichtung 2 umwandelt und/oder anpasst (siehe Figur 6).

Die Konstantstromquelle 23 kann darüber hinaus einen Shunt-Widerstand 25 umfassen (siehe Figur 6). Der Shunt-Widerstand 25 ist vorzugsweise zur Überwachung des Stroms für die Strahlungsemissionsvorrichtung 2, insbesondere für das zumindest eine optische Halbleiterelement, ausgebildet. Insbesondere verhindert der Shunt-Widerstand 25, dass die strahlungsemittierende Vorrichtung 2 mit einer zu hohen elektrischen Stromstärke versorgt wird.

Nun wird auf das Ausführungsbeispiel der Figur 6 Bezug genommen: Das medizinische oder dentale Behandlungsgerät 11 der Figur 6 besitzt einen einzigen Sensor 10, der über Daten- oder Signalleitungen 6D mit der medizinischen oder dentalen Instrumententeil-Steuerung 5B verbunden ist. Die Sensorsignale des einzigen Sensors 10 werden über die medizinische oder dentale Instrumententeil-Steuerung 5B und die Leitung 6C an die Steuer-, Regel- oder Versorgungseinheit 4, insbesondere den Mikrocontroller 19, übertragen. Die Sensorsignale des einzigen Sensors 10 können von dem medizinischen oder dentalen Instrumententeil-Steuerung 5B wie oben beschrieben gespeichert oder verarbeitet werden. Die medizinische oder dentale Instrumententeil-Steuerung 5B kann den Sensor 10 wie oben beschrieben mit elektrischer Energie versorgen, vorzugsweise über Leitungen 6D, die somit als gemeinsame Leitungen und/oder zur Übertragung von Sensorsignalen und elektrischer Energie ausgebildet sind.

Die Konstantstromquelle 23 versorgt die Strahlungsquelle 2 mit elektrischer Energie mit konstanter Stromstärke.

Mit Bezug auf Figur 7 erfolgt die Versorgung des Sensors 10 mit elektrischer Energie hier direkt, d.h. nicht über die medizinische oder dentale Instrumententeil-Steuerung 5B wie in Figur 6. Dem Sensor 10 ist eine Vorrichtung zur Aufbereitung elektrischer Energie 26 zugeordnet, wobei die Energie von der gemeinsamen elektrischen Energiequelle 12 über die gemeinsamen elektrischen Leitungen 6A, 6B zugeführt wird, um den Sensor 10 mit der von ihm benötigten elektrischen Energie zu versorgen. Die Vorrichtung zur Aufbereitung der elektrischen Leistung 26 umfasst beispielsweise einen DC/DC-Wandler.

Dementsprechend dient die Leitung 6D als reine Signal- oder Datenleitung zur Übertragung der Sensorsignale des Sensors 10 an den medizinischen oder dentalen Instrumententeil-Steuerung 5B. Die Sensorsignale des Sensors 10 werden über den medizinischen oder dentalen Instrumententeil-Steuerung 5B und die Leitung 6C an die Steuer-, Regel- oder Versorgungseinheit 4, insbesondere den Mikrocontroller 19, übertragen. Die Sensorsignale des Sensors 10 können von dem medizinischen oder dentalen Instrumententeil-Steuerung 5B wie oben beschrieben gespeichert oder verarbeitet werden.

Das medizinische oder dentale Behandlungsgerät 11 aus Figur 8 umfasst mehrere Sensoren 10. Bei den Sensoren 10 kann es sich um Sensoren desselben Typs und/oder um Sensoren handeln, die dieselbe Eigenschaft messen. Alternativ kann zumindest ein Sensor der mehreren Sensoren 10 ein Sensor eines anderen Typs sein und/oder ein Sensor, der eine andere Eigenschaft misst. Vorzugsweise ist jeder Sensor der mehreren Sensoren 10 von einem anderen Typ und/oder misst eine andere Eigenschaft.

Zumindest ein Sensor der mehreren Sensoren 10 wird direkt mit elektrischer Energie versorgt, d.h. nicht über die medizinische oder dentale Instrumententeil-Steuerung 5B wie in Figur 6. Dem zumindest einen Sensor 10 ist eine Vorrichtung zur Aufbereitung elektrischer Energie 26 zugeordnet, die von der gemeinsamen elektrischen Energiequelle 12 über die gemeinsamen elektrischen Leitungen 6A, 6B zugeführt wird, um den Sensor 10 mit der von ihm benötigten elektrischen Energie zu versorgen. Die Vorrichtung zur Verarbeitung der elektrischen Leistung 26 umfasst beispielsweise einen DC/DC-Wandler. Vorzugsweise werden mehrere oder alle Sensoren der Mehrzahl von Sensoren 10 direkt mit elektrischer Energie versorgt.

Alternativ wird zumindest ein Sensor aus der Vielzahl der Sensoren 10 über das medizinische oder dentale Instrumententeil-Steuerung 5B und die Leitung 6D mit elektrischer Energie versorgt, wie unter Bezugnahme auf Figur 6 beschrieben.

Die Leitung 6D dient als Signal- oder Datenleitung zur Übertragung der Sensorsignale zumindest eines Sensors oder mehrerer oder aller Sensoren der Vielzahl von Sensoren 10 an die medizinische oder dentale Instrumententeil-Steuerung 5B. Die Sensorsignale werden über die medizinischen oder dentalen Instrumententeil-Steuerung 5B und die Leitung 6C an die Steuer-, Regel- oder Versorgungseinheit 4, insbesondere den Mikrocontroller 19, übertragen. Die Sensorsignale können von dem medizinischen oder dentalen Instrumententeil-Steuerung 5B wie oben beschrieben gespeichert oder verarbeitet werden.

Zur Steuerung der elektromagnetischen Strahlungsemission der elektromagnetischen Strahlungsemissionsvorrichtung 2, z. B. der Wellenlänge oder Intensität, ist die medizinische oder dentale Instrumententeil-Steuerung 5B mit der elektrischen Konstantstromquelle 23, die der elektromagnetischen Strahlungsemissionsvorrichtung 2 zugeordnet ist, über elektrische Steuerleitungen 27 verbunden. Die medizinische oder dentale Instrumententeil-Steuerung 5B ist ausgebildet, Steuersignale über die Steuerleitungen 27 an die elektrische Konstantstromquelle 23 zu übermitteln, um die Emission elektromagnetischer Strahlung durch die elektromagnetische Strahlungsemissionsvorrichtung 2 zu steuern. Die elektrische Konstantstromquelle 23 der elektromagnetischen Strahlungsemissionsvorrichtung 2 ist ausgebildet, die Steuersignale zu empfangen und die elektromagnetische Strahlungsemissionsvorrichtung 2 entsprechend den empfangenen Steuersignalen zu betreiben.

Die elektromagnetische Strahlungsemissionsvorrichtung 2 kann eine mehrere Strahlungsemissionsquellen, z.B. LEDs, umfassen, wobei die medizinische oder dentale Instrumententeil-Steuerung 5B so ausgebildet sein kann, dass sie zumindest eine Strahlungsemissionsquelle der mehreren Strahlungsemissionsquellen steuert. Die mehreren Strahlungsquellen können vorzugsweise eine erste und eine zweite Strahlungsquelle umfassen, die eine erste und eine zweite, unterschiedliche Wellenlänge emittieren, insbesondere Wellenlängen für die Diagnose, z. B. für die Erkennung von Zahnkaries, Konkrement oder Plaque, und Wellenlängen zur Verbesserung der Sichtbarkeit für einen Benutzer, wobei die medizinische oder dentale Instrumententeil-Steuerung 5B ausgebildet ist, abwechselnd die Emission elektromagnetischer Strahlung der ersten oder zweiten Strahlungsquelle zu aktivieren.

Obwohl in den Figuren nicht dargestellt, kann die Steuerung der elektromagnetischen Strahlungsemission der elektromagnetische Strahlung emittierenden Vorrichtung 2 durch die medizinische oder dentale Instrumententeil-Steuerung 5B über elektrische Steuerleitungen 27 auch in den medizinischen oder dentalen Behandlungsvorrichtungen 11 der Figuren 6 und 7 realisiert werden.

Es wird nun auf die Figuren 9 bis 11 Bezug genommen. Diese Figuren zeigen Ausführungsbeispiele von medizinischen oder dentalen Behandlungsgeräten 11 gemäß der beanspruchten Erfindung mit mehreren medizinischen oder dentalen Instrumententeilen 1, 1C, 1D. Die medizinische oder dentale Behandlungsvorrichtung 11 kann eine unterschiedliche Anzahl von medizinischen oder dentalen Instrumententeilen 1, 1C, 1D umfassen, beispielsweise zwei, drei, vier, fünf oder mehr. Diese medizinischen oder dentalen Instrumententeile 1, 1C, 1D sind oder können miteinander, vorzugsweise lösbar, verbunden werden, um eine medizinische oder dentale Behandlungsvorrichtung 11 zu bilden. Eine physische Ansicht einer solchen medizinischen oder dentalen Behandlungsvorrichtung 11 ist in Figur 1 dargestellt.

Die in den Figuren 3, 4 und 6 bis 8 gezeigten Elemente sind auch in den Figuren 9 bis 11 dargestellt und mit denselben Ziffern versehen. Alle Merkmale, Funktionen oder Eigenschaften, die oben im Zusammenhang mit diesen Elementen erörtert wurden, können auch für die in den Figuren 9 bis 11 dargestellten Elemente gelten.

Die medizinischen oder dentalen Instrumententeile 1, 1C, 1D sind, insbesondere wenn sie miteinander verbunden sind, über den Versorgungsschlauch 15 mit der (gemeinsamen) Steuer-, Regel- oder Versorgungseinheit 4 verbunden, um zumindest eines der folgenden Mittel zu übertragen oder auszutauschen: elektrische Energie; mechanische Energie; ein Fluid; ein Medium; einen Stoff; Signale oder Daten. Leitungen und/oder Rohre zur Übertragung, insbesondere elektrische Leitungen 6A, 6B, 6C, reichen also von der Steuer-, Regel- oder Versorgungseinheit 4 durch den Versorgungsschlauch 15 zu zumindest einem der medizinischen oder dentalen Instrumententeile 1, 1C, 1D. Bei den Leitungen kann es sich um optische oder elektrische Leitungen handeln.

Alternativ reichen Leitungen und/oder Rohre zur Übertragung, insbesondere elektrische Leitungen 6A, 6B, 6C, von der Steuer-, Regel- oder Versorgungseinheit 4 durch den Versorgungsschlauch 15 zu mehreren oder allen medizinischen oder dentalen Instrumententeilen 1, 1C, 1D.

Die elektrischen Leitungen 6A, 6B, 6C sind vorzugsweise Teil eines Bussystems, insbesondere eines CAN-, Flex Ray-, Lin-, SPI- oder I²C-Bussystems, wobei mehrere Komponenten der medizinischen oder dentalen Instrumententeile 1, 1C, 1D, die zur Aufnahme von elektrischer Energie und/oder zur Kommunikation untereinander und/oder mit einer (gemeinsamen) Steuer-, Regel- oder Versorgungseinheit 4 ausgebildet sind, über die elektrischen Leitungen 6A, 6B, 6C verbunden sind und kommunizieren und/oder mit Energie versorgt werden. Die mehreren Komponenten der medizinischen oder dentalen Instrumententeile 1, 1C, 1D können beispielsweise eine oder mehrere elektromagnetische Strahlung emittierende Vorrichtungen 2, eine oder mehrere medizinische oder dentale Instrumententeil-Steuerungen 5B, ein oder mehrere Speicherelemente 5A, einen oder mehrere Sensoren 10 umfassen. Die Komponenten dieser mehreren Komponenten können entweder in einem einzigen medizinischen oder dentalen Instrumententeil 1, 1C, 1D oder in mehreren medizinischen oder dentalen Instrumententeilen 1, 1C, 1D angeordnet sein, die zur Bildung von medizinischen oder dentalen Behandlungsgeräten 11 verbunden sind oder verbunden werden können.

Gemäß einem besonders bevorzugten Ausführungsbeispiel kann das medizinische oder dentale Instrumententeil 1 ein medizinisches oder dentales Handstück 17, insbesondere wie oben beschrieben, umfassen, das medizinische oder dentale Instrumententeil 1C kann eine medizinische oder dentale Antriebseinheit oder einen Elektromotor 14, insbesondere wie oben beschrieben, umfassen, und das medizinische oder dentale Instrumententeil 1D kann eine medizinische oder dentale Kupplungsvorrichtung umfassen. Die medizinische oder dentale Kupplungsvorrichtung 1D kann entweder einstückig mit dem Versorgungsschlauch 15 ausgebildet oder ein separates Teil sein, das lösbar mit dem Versorgungsschlauch 15 verbunden ist.

Es wird jedoch ausdrücklich darauf hingewiesen, dass zumindest eines der medizinischen oder dentalen Instrumententeile 1, 1C, 1D eine andere Funktion und/oder zumindest eine andere Komponente aufweisen kann. Die medizinische oder dentale Behandlungsvorrichtung 11 kann beispielsweise ein medizinisches oder dentales Instrumententeil 1 mit einer elektromagnetischen Strahlungsemissionsvorrichtung 2 und einem optischen Sensor 10, insbesondere einer Kamera, und ein medizinisches oder dentales Instrumententeil 1C oder 1D mit zumindest einem weiteren Sensor 10 umfassen.

Das medizinische oder dentale Instrumententeil 1 der Figur 9 und das medizinische oder dentale Instrumententeil 1 der Figur 10 umfassen Komponenten, die oben beschrieben wurden oder Komponenten entsprechen, die oben beschrieben wurden: Strahlungsemissionsvorrichtung oder Beleuchtungsvorrichtung 2; medizinische oder dentale Instrumententeil-Steuerung oder Mikrocontroller 5B; elektrische Leitungen 6A, 6B, 6C; Sensor 10; erste Vorrichtung zur Aufbereitung elektrischer Energie 21; zweite Vorrichtung zur Aufbereitung elektrischer Energie 22; elektrische Konstantstromquelle 23 der elektromagnetischen Strahlungsemissionsvorrichtung 2 mit Elektronik 24 und Shunt-Widerstand 25; elektrische Steuerleitungen 27. Der Sensor 10 ist über die Leitung 6D mit der Steuerung für medizinische oder dentale Instrumente oder dem Mikrocontroller 5B verbunden, wobei die Leitung 6D je nach Art des Sensors entweder nur zur Übertragung von Sensorsignalen oder zusätzlich zur Übertragung von elektrischer Energie an den Sensor 10 ausgebildet sein kann.

Das medizinische oder dentale Instrumententeil 1C der Figur 9 und das medizinische oder dentale Instrumententeil 1C der Figur 10 umfassen Komponenten, die oben beschrieben wurden oder Komponenten entsprechen, die oben beschrieben wurden: medizinische oder dentale Instrumententeil-Steuerung oder Mikrocontroller 5C; elektrische Leitungen 6A, 6B, 6C; Sensor 10C; zumindest eine Vorrichtung zur Aufbereitung elektrischer Energie 21, 22. Der Sensor 10C ist über die Leitung 6D mit der Steuerung oder dem Mikrocontroller 5C für medizinische oder dentale Instrumente verbunden, wobei die Leitung 6D je nach Art des Sensors entweder nur zur Übertragung von Sensorsignalen oder zusätzlich zur Übertragung von elektrischer Energie an den Sensor 10 ausgebildet sein kann.

Das medizinische oder dentale Instrumententeil 1D der Figur 9 umfasst eine Kupplungsvorrichtung, die vorzugsweise einteilig mit dem Versorgungsschlauch 15 ausgebildet ist. Gemäß diesem Ausführungsbeispiel umfasst das medizinische oder dentale Instrumententeil 1D nur die elektrischen Leitungen 6A, 6B, 6C.

Das medizinische oder dentale Instrumententeil 1D der Figur 10 umfasst eine Kupplungsvorrichtung, die entweder einstückig mit dem Versorgungsschlauch 15 ausgebildet sein kann oder eine separate Vorrichtung darstellt, die vom Versorgungsschlauch 15 lösbar ist. Das medizinische oder dentale Instrumententeil 1D der Figur 10 umfasst Komponenten, die oben beschrieben wurden oder den oben beschriebenen Komponenten entsprechen: Medizinische oder dentale Instrumententeil-Steuerung oder Mikrocontroller 5D; elektrische Leitungen 6A, 6B, 6C; mehrere Sensoren 10D; zumindest eine Vorrichtung zur Aufbereitung elektrischer Energie 21, 22. Die Sensoren 10D sind über die Leitungen 6D mit der medizinischen oder dentalen Instrumenten-Steuerung oder Mikrocontroller 5D verbunden, wobei jede entsprechende Leitung 6D so ausgebildet sein kann, dass sie je nach Art des Sensors entweder nur Sensorsignale oder zusätzlich elektrische Energie an einen Sensor der mehreren Sensoren 10 überträgt.

Das medizinische oder dentale Instrumententeil 1 der Figur 11 umfasst Komponenten, die oben beschrieben wurden oder Komponenten entsprechen, die oben beschrieben wurden: medizinische oder dentale Instrumententeil-Steuerung oder Mikrocontroller 5B; elektrische Leitungen 6A, 6B, 6C; mehrere Sensoren 10; zumindest eine Vorrichtung zur Aufbereitung elektrischer Energie 21, 22. Die Sensoren 10D sind über die Leitungen 6D mit der medizinischen oder dentalen Instrumenten-Steuerung oder dem Mikrocontroller 5D verbunden, wobei jede Leitung 6D so ausgebildet sein kann, dass sie je nach Art des Sensors entweder nur Sensorsignale oder zusätzlich elektrische Energie an einen Sensor der mehreren Sensoren 10 überträgt.

Das medizinische oder dentale Instrumententeil 1C der Figur 11 umfasst Komponenten, die oben beschrieben wurden oder den oben beschriebenen Komponenten entsprechen: Strahlungsemissionsvorrichtung oder Beleuchtungsvorrichtung 2; medizinische oder dentale Instrumententeil-Steuerung oder Mikrocontroller 5C; elektrische Leitungen 6A, 6B, 6C; Sensor 10; eine erste Vorrichtung zur Aufbereitung elektrischer Energie 21; eine zweite Vorrichtung zur Aufbereitung elektrischer Energie 22; elektrische Konstantstromquelle 23 der elektromagnetischen Strahlungsemissionsvorrichtung 2 mit Elektronik 24 und Shunt-Widerstand 25; elektrische Steuerleitungen 27. Der Sensor 10 ist über die Leitung 6D mit der Steuereinheit oder dem Mikrocontroller 5C für medizinische oder dentale Instrumente verbunden, wobei die Leitung 6D je nach Art des Sensors entweder nur zur Übertragung von Sensorsignalen oder zusätzlich zur Übertragung von elektrischer Energie an den Sensor 10 ausgebildet sein kann.

Das medizinische oder dentale Instrumententeil 1D der Figur 11 umfasst Komponenten, die oben beschrieben wurden oder Komponenten entsprechen, die oben beschrieben wurden: medizinische oder dentale Instrumententeil-Steuerung oder Mikrocontroller 5D; elektrische Leitungen 6A, 6B, 6C; Sensor 10D; zumindest eine Vorrichtung zur Aufbereitung elektrischer Energie 21, 22. Der Sensor 10D ist über die Leitung 6D mit der medizinischen oder dentalen Instrumententeil-Steuerung oder Mikrocontroller 5D für verbunden, wobei die Leitung 6D je nach Art des Sensors entweder nur zur Übertragung von Sensorsignalen oder zusätzlich zur Übertragung von elektrischer Energie an den Sensor 10 ausgebildet sein kann.

Das medizinische oder dentale Instrumententeil 1 und/oder 1C aus Figur 11 kann einen optischen Strahlungs- oder Lichtleiter umfassen, um die von der elektromagnetische Strahlung emittierenden Vorrichtung 2 ausgesandte elektromagnetische Strahlung durch das medizinische oder dentale Instrumententeil 1 und/oder 1C zu leiten. Somit kann die elektromagnetische Strahlung vom Instrumententeil 1 abgestrahlt werden.

In den Figuren 9 - 11 ist in jedem medizinischen oder dentalen Instrumententeil 1, 1C und 1D eine Vorrichtung zur Aufbereitung elektrischer Energie 21 vorgesehen. Alternativ ist es auch möglich, nur eine einzige derartige Vorrichtung 21 pro medizinischem oder dentalem Behandlungsgerät 11 vorzusehen, welche die von der Stromquelle 12 erhaltene elektrische Energie aufbereitet und die aufbereitete elektrische Energie über die elektrischen Leitungen 6A, 6B an alle Komponenten des medizinischen oder dentalen Behandlungsgeräts 11 liefert, die mit elektrischer Energie versorgt werden, insbesondere an die Komponenten 2, 5B, 5C, 5D und 10. Eine solche einzelne Vorrichtung 21 ist vorzugsweise in der Nähe der Versorgungsleitung 15, insbesondere im medizinischen oder dentalen Instrumententeil 1D, angeordnet.

Die elektrischen Leitungen 6A, 6B, 6C der Figuren 9 - 11 verlaufen von der (gemeinsamen) Steuer-, Regel- oder Versorgungseinheit 4 durch den Versorgungsschlauch 15 und jedes der medizinischen oder dentalen Instrumententeile 1, 1C, 1D. Die Komponenten 2; 5B; 10; 21; 22; 23; 5C; 10C; 5D, 10D sind mit den elektrischen Leitungen 6A, 6B, 6C verbunden, vorzugsweise über elektrische Abzweigleitungen, und erhalten elektrische Energie und/oder kommunizieren über die elektrischen Leitungen 6A, 6B, 6C.

Die Kommunikation über die elektrischen Leitungen 6A, 6B, 6C kann zumindest eines der folgenden Elemente umfassen: Übermittlung von Signalen der Sensoren 10, 10C, 10D an die Steuer-, Regel- oder Versorgungseinheit 4; Übermittlung von Signalen von der Steuer-, Regel- oder Versorgungseinheit 4 an zumindest einen der Sensoren 10, 10C, 10D, insbesondere wenn zumindest einer der Sensoren 10, 10C, 10D einen digitalen Sensor umfasst; Übermittlung von Identifikations- und/oder Betriebs- und/oder Pflegedaten von zumindest einer der medizinischen oder dentalen Instrumententeil-Steuerungen 5B, 5C, 5D oder dem Speicherelement 5A an die Steuer-, Regel- oder Versorgungseinheit 4; Übertragung von Daten von der Steuer-, Regel- oder Versorgungseinheit 4 an zumindest eine der medizinischen oder dentalen Instrumententeil-Steuerungen 5B, 5C, 5D oder das Speicherelement 5A; Übertragung von Signalen oder Daten zwischen zumindest zwei dentalen Instrumententeil-Steuerungen 5B, 5C, 5D; Übertragung von Signalen oder Daten zwischen zumindest zwei Sensoren 10, 10C, 10D, insbesondere wenn einer der Sensoren 10, 10C, 10D einen virtuellen Sensor umfasst.

Zur Durchführung der oben beschriebenen elektrischen Energieübertragung und Kommunikation bilden die elektrischen Leitungen 6A, 6B, 6C, Sensoren 10, 10C, 10D, medizinische oder dentale Instrumententeil-Steuerungen 5B, 5C, 5D, Speicherelement 5A und Steuer-, Regel- oder Versorgungseinheit 4, insbesondere Mikrocontroller 19, ein Bussystem, insbesondere ein Bussystem wie oben beschrieben.

## Patentansprüche

1. Medizinisches oder dentales Instrumententeil (1), umfassend: eine Kupplungsvorrichtung (3), welche das medizinische oder dentale Instrumententeil (1) mit einem Versorgungsschlauch (15) zum Anschluss des medizinischen oder zahnmedizinischen Instrumententeils (1) an eine Steuer-, Regel- oder Versorgungseinheit (4) verbindet, eine mit elektrischer Energie betreibbare, medizinische oder dentale Instrumententeil-Steuerung (5B), zumindest einen Sensor (10) zur Erzeugung eines Sensorsignals, und drei elektrische Leitungen (6A, 6B, 6C), wobei eine erste gemeinsame elektrische Leitung (6A) und eine zweite gemeinsame elektrische Leitung (6B) der drei elektrischen Leitungen (6A, 6B, 6C) mit der medizinischen oder dentalen Instrumententeil-Steuerung (5B) und einer am medizinischen oder dentalen Instrumententeil (1) angeordneten oder mit dem medizinischen oder dentalen Instrumententeil (1) verbindbaren, elektromagnetische Strahlung emittierenden Vorrichtung (2) verbunden sind, um elektrische Energie zum Betrieb der medizinischen oder dentalen Instrumententeil-Steuerung (5B) und der elektromagnetische Strahlung emittierenden Vorrichtung (2) von einer gemeinsamen elektrischen Energiequelle (12) zu liefern, und wobei eine dritte elektrische Leitung (6C) der drei elektrischen Leitungen (6A, 6B, 6C) die medizinische oder dentale Instrumententeil-Steuerung (5B) mit der Steuer-, Regel- oder Versorgungseinheit (4) verbindet, wobei
der zumindest eine Sensor (10) mit der dritten elektrischen Leitung (6C) der drei elektrischen Leitungen (6A, 6B, 6C) verbunden ist, so dass das Sensorsignal an die Steuer-, Regel- oder Versorgungseinheit (4) übertragbar ist, **gekennzeichnet durch** eine als DC/DC-Wandler ausgebildete erste Vorrichtung zur Aufbereitung elektrischer Energie (21, 22, 26), die ausgebildet ist, die von der gemeinsamen elektrischen Energiequelle (12) über die erste und zweite gemeinsame elektrische Leitung (6A, 6B) an das medizinische oder zahnmedizinische Instrumententeil (1) gelieferte elektrische Energie für zumindest zwei der folgenden Elemente: der medizinischen oder dentalen Instrumententeil-Steuerung (5B); dem zumindest einen Sensor (10); der elektromagnetische Strahlung emittierenden Vorrichtung (2) aufzubereiten, in dem der DC/DC-Wandler das Spannungsniveau der elektrischen Leistung der elektrischen Stromquelle (12) in ein anderes Spannungsniveau umwandelt.

2. Medizinisches oder dentales Instrumententeil (1) nach Anspruch 1, **dadurch gekennzeichnet, dass**
der zumindest eine Sensor (10) mit der medizinischen oder dentalen Instrumententeil-Steuerung (5B) verbunden ist, so dass das Sensorsignal des zumindest einen Sensors (10) über die medizinische oder dentale Instrumententeil-Steuerung (5B) und die dritte elektrische Leitung (6C) an die Steuer-, Regel- oder Versorgungseinheit (4) übertragbar ist.

3. Medizinisches oder dentales Instrumententeil (1) nach Anspruch 2, **dadurch gekennzeichnet, dass**
die medizinische oder dentale Instrumententeil-Steuerung (5B) ausgebildet ist, das Sensorsignal des zumindest einen Sensors (10) zu verarbeiten und/ oder zu speichern.

4. Medizinisches oder dentales Instrumententeil (1) nach Anspruch 2 oder 3, **dadurch gekennzeichnet, dass**
die medizinische oder dentale Instrumententeil-Steuerung (5B) basierend auf dem Sensorsignal zumindest eine Komponente des medizinischen oder dentalen Instrumententeils (1) betreibt oder regelt oder steuert.

5. Medizinisches oder dentales Instrumententeil (1) nach einem der vorstehenden Ansprüche, **dadurch gekennzeichnet, dass**
die medizinische oder dentale Instrumententeil-Steuerung (5B) ein Speicherelement (5) zum Speichern von Identifikationsdaten und/oder Betriebsdaten und/oder Pflegedaten des medizinischen oder dentalen Instrumententeils (1) aufweist, wobei das Speicherelement (5) mit der dritten elektrischen Leitung (6C) verbunden ist, so dass die Identifikationsdaten und/oder Betriebsdaten und/oder Pflegedaten des medizinischen oder dentalen Instrumententeils (1) über die dritte elektrische Leitung (6C) zwischen der Steuer-, Regel- oder Versorgungseinheit (4) und dem Speicherelement (5) übertragbar sind.

6. Medizinisches oder dentales Instrumententeil (1) nach einem der vorstehenden Ansprüche, **gekennzeichnet durch**
eine zweite Vorrichtung zur Aufbereitung elektrischer Energie (21, 22, 26), die ausgebildet ist, die von der gemeinsamen elektrischen Energiequelle (12) über die erste und zweite gemeinsame elektrische Leitung (6A, 6B) an das medizinische oder zahnmedizinische Instrumententeil (1) gelieferte elektrische Energie aufzubereiten.

7. Medizinisches oder dentales Instrumententeil (1) nach einem der vorstehenden Ansprüche, **gekennzeichnet durch**
eine elektrische Konstantstromquelle (23), die der elektromagnetische Strahlung emittierenden Vorrichtung (2) zugeordnet ist und ausgebildet ist, die elektromagnetische Strahlung emittierenden Vorrichtung (2) mit elektrischer Energie mit einer konstanten Stromstärke zu versorgen, wobei die elektrische Konstantstromquelle (23) über die erste und zweite gemeinsame elektrische Leitung (6A, 6B) mit der gemeinsamen elektrischen Energiequelle (12) verbunden ist, um elektrische Energie von der gemeinsamen elektrischen Energiequelle (12) zu empfangen, um die elektromagnetische Strahlung emittierende Vorrichtung (2) zu betreiben.

8. Medizinisches oder dentales Instrumententeil (1) nach einem der vorstehenden Ansprüche, **dadurch gekennzeichnet, dass**
die medizinische oder dentale Instrumententeil-Steuerung (5B) ausgebildet ist, die Abgabe von elektromagnetischer Strahlung der elektromagnetische Strahlung emittierenden Vorrichtung (2) zu steuern.

9. Medizinisches oder dentales Instrumententeil (1) nach einem der vorstehenden Ansprüche, **dadurch gekennzeichnet, dass**
die erste und zweite gemeinsame elektrische Leitung (6A, 6B) den zumindest einen Sensor (10) mit der gemeinsamen elektrischen Energiequelle (12) verbindet, um den zumindest einen Sensor (10) mit elektrischer Energie zu versorgen.

10. Medizinisches oder dentales Instrumententeil (1) nach einem der vorstehenden Ansprüche, **dadurch gekennzeichnet, dass**
die medizinische oder dentale Instrumententeil-Steuerung (5B) ausgebildet ist, den zumindest einen Sensor (10) mit elektrischer Energie zu versorgen.

11. Medizinisches oder dentales Instrumententeil (1) nach einem der vorstehenden Ansprüche, **dadurch gekennzeichnet, dass**
der zumindest eine Sensor (10) ein Accelerometer oder ein Gyroskop umfasst.

12. Medizinisches oder dentales Instrumententeil (1) nach einem der vorstehenden Ansprüche, **dadurch gekennzeichnet, dass**
der zumindest eine Sensor (10) einen Temperatursensor zur Messung der Temperatur zumindest einer Komponente des medizinischen oder dentalen Instrumententeils (1) oder zumindest einer an dem medizinischen oder dentalen Instrumententeil (1) befestigten Komponente oder eines durch das medizinische oder dentale Instrumententeil (1) bearbeitetes Werkstück aufweist.

13. Medizinische oder zahnärztliche Behandlungsvorrichtung (11), **gekennzeichnet durch** ein erstes medizinisches oder dentales Instrumententeil (1) nach einem der vorstehenden Ansprüche,
eine gemeinsame elektrische Energiequelle (12) zur Versorgung des medizinischen oder zahnmedizinischen Instrumententeils (1) mit elektrischer Energie, und
eine Steuer-, Regel- oder Versorgungseinheit (4) zum Empfang des Sensorsignals des zumindest einen Sensors (10).

14. Medizinische oder zahnärztliche Behandlungsvorrichtung (11) nach Anspruch 13, **gekennzeichnet, durch**
ein zweites medizinisches oder dentales Instrumententeil (1C, 1D), wobei das erste medizinische oder dentale Instrumententeil (1) und das zweite medizinische oder dentale Instrumententeil (1C, 1D) miteinander verbindbar sind, um über einen gemeinsamen Versorgungsschlauch (15) mit der gemeinsamen elektrischen Energiequelle (12) und einer Steuer-, Regel- oder Versorgungseinheit (4) verbunden zu sein, wobei die erste und zweite gemeinsame elektrische Leitung (6A, 6B) der drei elektrischen Leitungen (6A, 6B, 6C) elektrische Energie von der gemeinsamen elektrischen Energiequelle (12) an die elektromagnetische Strahlung emittierende Vorrichtung (2) zum Betrieb der elektromagnetische Strahlung emittierenden Vorrichtung (2) und an die medizinische oder dentale Instrumententeil-Steuerung (5B) des erstes medizinisches oder dentales Instrumententeil (1) zum Betrieb derselben übertragen, und wobei die dritte elektrische Leitung (6C) der drei elektrischen Leitungen (6A, 6B, 6C) mit dem zumindest einen Sensor (10) verbunden ist, so dass die Sensorsignale des zumindest einen Sensors (10) über die dritte elektrische Leitung (6C) an die gemeinsame Steuer-, Regel- oder Versorgungseinheit (4) übertragbar sind.

15. Medizinische oder zahnärztliche Behandlungsvorrichtung (11) nach Anspruch 13 oder 14, **dadurch gekennzeichnet, dass**
die gemeinsame elektrische Energiequelle (12) eine Konstantspannungsquelle aufweist oder die gemeinsame elektrische Energiequelle (12) ist ausgebildet, Gleichstrom zur Verfügung zu stellen.

## Claims

1. A medical or dental instrument part (1), comprising: a coupling device (3) which couples the medical or dental instrument part (1) to a supply tube (15) for connection of the medical or dental instrument part (1) to a control, regulating or supply unit (4), a medical or dental instrument part controller (5B) operable with electrical power, at least one sensor (10) for generating a sensor signal, and three electrical lines (6A, 6B, 6C), wherein a first shared electric line (6A) and a second shared electric line (6B) of the three electrical lines (6A, 6B, 6C) connect to the medical or dental instrument part controller (5B) and to an electromagnetic radiation emitting device (2) that is provided on the medical or dental instrument part (1) or can be connected to the medical or dental instrument part (1) to supply electrical power from a shared electrical power source (12) to operate the dental instrument part controller (5B) and the electromagnetic radiation emitting device (2), and wherein a third electrical line (6C) of the three electrical lines (6A, 6B, 6C) connects the medical or dental instrument part controller (5B) to the control, regulation or supply unit (4), wherein
the at least one sensor (10) connects to the third electric line (6C) of the three electric lines (6A, 6B, 6C), so that the sensor signal can be transmitted to the control, regulating or supply unit (4), **characterized by**
a first device for processing electrical energy (21, 22, 26) designed as a DC/DC converter, which is configured to process the electrical energy supplied from the shared electrical energy source (12) via the first and second shared electrical lines (6A, 6B) to the medical or dental instrument part (1) for at least two of the following elements: the medical or dental instrument part controller (5B); the at least one sensor (10); the electromagnetic radiation emitting device (2), wherein the DC/DC converter converts the voltage level of the electrical power of the electrical power source (12) into another voltage level.

2. The medical or dental instrument part (1) according to claim 1, **characterized in that** the at least one sensor (10) connects to the medical or dental instrument part controller (5B) so that the sensor signal of the at least one sensor (10) can be transmitted via the medical or dental instrument part controller (5B) and the third electric line (6C) to the control, regulating or supply unit (4).

3. The medical or dental instrument part (1) according to claim 2, **characterized in that** the medical or dental instrument part controller (5B) is configured to process and/or store the sensor signal of the at least one sensor (10).

4. The medical or dental instrument part (1) according to claim 2 or 3, **characterized in that**
the medical or dental instrument part controller (5B) operates or regulates or controls at least one component of the medical or dental instrument part (1) based on the sensor signal.

5. The medical or dental instrument part (1) according to any one of the preceding claims, **characterized in that**
the medical or dental instrument part controller (5B) comprises a memory element (5) for storing identification data and/or operating data and/or care data of the medical or dental instrument part (1), wherein the memory element (5) connects to the third electric line (6C) so that the identification data and/or operating and/or care data of the medical or dental instrument part (1) can be transmitted over the third electric line (6C) between the control, regulating or supply unit (4) and the memory element (5).

6. The medical or dental instrument part (1) according to any one of the preceding claims, **characterized by**
a second device (21, 22, 26) for processing electrical power which is configured to process the electrical power supplied by the shared electrical power source (12) via the first or second shared electric line (6A, 6B) to the medical or dental instrument part (1).

7. The medical or dental instrument part (1) according to any one of the preceding claims, **characterized by**
an electrical constant current source (23) which is assigned to the electromagnetic radiation emitting device (2) and configured to supply the electromagnetic radiation emitting device (2) with electrical power having a constant amperage, wherein the electrical constant current source (23) connects via the first and second shared electric lines (6A, 6B) to the shared electrical power source (12) to receive electrical power from the shared electrical power source (12) to operate the electromagnetic radiation emitting device (2).

8. The medical or dental instrument part (1) according to any one of the preceding claims, **characterized in that**
the medical or dental instrument part controller (5B) is configured to control the electromagnetic radiation emission of the electromagnetic radiation emitting device (2).

9. The medical or dental instrument part (1) according to any one of the preceding claims, **characterized in that**
the first and second shared electric lines (6A, 6B) connect the at least one sensor (10) to the shared electrical power source (12) to supply electrical power to the at least one sensor (10).

10. The medical or dental instrument part (1) according to any one of the preceding claims, **characterized in that**
the medical or dental instrument part controller (5B) is configured to supply electrical power to the at least one sensor (10).

11. The medical or dental instrument part (1) according to any one of the preceding claims, **characterized in that**
the at least one sensor (10) comprises an accelerometer or a gyroscope.

12. The medical or dental instrument part (1) according to any one of the preceding claims, **characterized in that**
the at least one sensor (10) comprises a temperature sensor for measuring the temperature of at least one component of the medical or dental instrument part (1) or of at least one component attached to the medical or dental instrument part (1) or of a workpiece worked by the medical or dental instrument part (1).

13. A medical or dental treatment device (11), **characterized by**
a first medical or dental instrument part (1) according to any one of the preceding claims,
a shared electrical power source (12) for supplying electrical power to the medical or dental instrument part (1), and
a control, regulating or supply unit (4) for receiving the sensor signal of the at least one sensor (10).

14. The medical or dental treatment device (11) according to claim 13, **characterized by** a second medical or dental instrument part (1C, 1D), wherein the first medical or dental instrument part (1) and the second medical or dental instrument part (1C, 1D) are connectable to each other to be connected via a common supply hose (15) to the common electric power source (12) and to a control, regulating or supply unit (4), wherein the first and second common electric lines (6A, 6B) of the three electric lines (6A, 6B, 6C) transmit electrical power from the common electrical power source (12) to the electromagnetic radiation emitting device (2) for operating the electromagnetic radiation emitting device (2) and to the medical or dental instrument part controller (5B) of the first medical or dental instrument part (1) for operating the same, and wherein the third electrical line (6C) of the three electrical lines (6A, 6B, 6C) is connected to the at least one sensor (10), so that the sensor signals of the at least one sensor (10) can be transmitted via the third electrical line (6C) to the common control, regulation or supply unit (4).

15. The medical or dental treatment device (11) according to claim 13 or 14, **characterized in that**
the shared electrical power source (12) comprises a constant voltage source or the shared electrical power source (12) is configured to provide direct current.

## Revendications

1. Pièce d'instrument (1) médical ou dentaire, comprenant: un dispositif d'accouplement (3), lequel connecte la pièce d'instrument (1) médical ou dentaire avec un flexible d'alimentation (15) pour le raccordement de la pièce d'instrument (1) médical ou dentaire sur une unité de commande, de réglage ou d'alimentation (4), un système de commande (5B) de pièce d'instrument médical ou dentaire fonctionnant à l'énergie électrique, au moins un capteur (10), destiné à générer un signal de capteur, et trois conduites électriques (6A, 6B, 6C), une première conduite électrique (6A) commune et une deuxième conduite électrique (6B) commune des trois conduites électriques (6A, 6B, 6C) étant connectées avec le système de commande (5B) de pièce d'instrument médical ou dentaire et avec un dispositif (2) émettant un rayonnement électromagnétique placé sur la pièce d'instrument (1) médical ou dentaire ou susceptible de se connecter sur la pièce d'instrument (1) médical ou dentaire, pour fournir de l'énergie électrique d'une source d'énergie (12) électrique commune, pour le fonctionnement du système de commande (5B) de pièce d'instrument médical ou dentaire et du dispositif (2) émettant un rayonnement électromagnétique, et une troisième conduite électrique (6C) des trois conduites électriques (6A, 6B, 6C) connectant le système de commande (5B) de pièce d'instrument médical ou dentaire avec l'unité de commande, de réglage ou d'alimentation (4),
l'au moins un capteur (10) étant connecté avec la troisième conduite électrique (6C) des trois conduites électriques (6A, 6B, 6C), de telle sorte que le signal de capteur soit transmissible à l'unité de commande, de réglage ou d'alimentation (4), **caractérisée par** un premier dispositif (21, 22, 26) conçu sous la forme d'un convertisseur DC / DC, destiné à préparer de l'énergie électrique, qui est conçu pour préparer l'énergie électrique de la source d'énergie (12) électrique commune fournie par l'intermédiaire de la première et de le deuxième conduites électriques (6A, 6B) communes à la pièce d'instrument (1) médical ou dentaire pour l'au moins deux des éléments suivants: le système de commande (5B) de pièce d'instrument médical ou dentaire; l'au moins un capteur (10); du dispositif (2) émettant un rayonnement électromagnétique, en ce que le convertisseur DC / DC convertit le niveau de tension de la puissance électrique de la source d'énergie (12) électrique commune en un autre niveau de tension.

2. Pièce d'instrument (1) médical ou dentaire selon la revendication 1, **caractérisée en ce que**
l'au moins un capteur (10) est connecté avec le système de commande (5B) de pièce d'instrument médical ou dentaire, de telle sorte que le signal de capteur de l'au moins un capteur (10) soit transmissible par l'intermédiaire du système de commande (5B) de pièce d'instrument médical ou dentaire et de la troisième conduite électrique (6C) à l'unité de commande, de réglage ou d'alimentation (4).

3. Pèce d'instrument (1) médical ou dentaire selon la revendication 2, **caractérisée en ce que**
le système de commande (5B) de pièce d'instrument médical ou dentaire est conçu pour traiter et / ou pour mémoriser le signal de capteur de l'au moins un capteur (10) .

4. Pièce d'instrument (1) médical ou dentaire selon la revendication 2 ou 3, **caractérisée en ce que**
sur la base du signal de capteur, le système de commande (5B) de pièce d'instrument médical ou dentaire fait fonctionner ou règle ou commande au moins un composant de la pièce d'instrument (1) médical ou dentaire.

5. Pièce d'instrument (1) médical ou dentaire selon l'une quelconque des revendications précédentes, **caractérisée en ce que**
le système de commande (5B) de pièce d'instrument médical ou dentaire comporte un élément de mémoire (5), destiné à mémoriser des données d'identification et / ou des données opérationnelles et / ou des données d'entretien de la pièce d'instrument (1) médical ou dentaire, l'élément de mémoire (5) étant connecté avec la troisième conduite électrique (6C), de telle sorte que les données d'identification et / ou les données opérationnelles et / ou les données d'entretien de la pièce d'instrument (1) médical ou dentaire soient transmissibles par l'intermédiaire de la troisième conduite électrique (6C) entre l'unité de commande, de réglage ou d'alimentation (4) et l'élément de mémoire (5).

6. Pièce d'instrument (1) médical ou dentaire selon l'une quelconque des revendications précédentes, **caractérisée par**
un deuxième dispositif de préparation d'énergie (21, 22, 26) électrique, qui est conçu pour préparer l'énergie électrique fournie par la source d'énergie (12) électrique commune par l'intermédiaire de la première et de la deuxième conduites électriques (6A, 6B) communes à la pièce d'instrument (1) médical ou dentaire.

7. Pièce d'instrument (1) médical ou dentaire selon l'une quelconque des revendications précédentes, **caractérisée par**
une source électrique (23) de courant constant, qui est affectée au dispositif (2) émettant un rayonnement électromagnétique et qui est conçue pour alimenter le dispositif (2) émettant un rayonnement électromagnétique en énergie électrique à intensité de courant constante, la source électrique (23) de courant constant étant connectée par l'intermédiaire de la première et de la deuxième conduites électriques (6A, 6B) communes avec la source d'énergie (12) électrique commune, pour réceptionner de l'énergie électrique de la part de la source d'énergie (12) électrique commune, pour faire fonctionner le dispositif (2) émettant un rayonnement électromagnétique.

8. Pièce d'instrument (1) médical ou dentaire selon l'une quelconque des revendications précédentes, **caractérisée en ce que**
le système de commande (5B) de pièce d'instrument médical ou dentaire est conçu pour commander la distribution d'un rayonnement électromagnétique du dispositif (2) émettant un rayonnement électromagnétique.

9. Pièce d'instrument (1) médical ou dentaire selon l'une quelconque des revendications précédentes, **caractérisée en ce que**
la première et la deuxième conduites électriques (6A, 6B) communes connectent l'au moins un capteur (10) avec la source d'énergie (12) électrique commune, pour alimenter l'au moins un capteur (10) avec de l'énergie électrique.

10. Pièce d'instrument (1) médical ou dentaire selon l'une quelconque des revendications précédentes, **caractérisée en ce que**
le système de commande (5B) de pièce d'instrument médical ou dentaire est conçu pour alimenter l'au moins un capteur (10) avec de l'énergie électrique.

11. Pièce d'instrument (1) médical ou dentaire selon l'une quelconque des revendications précédentes, **caractérisée en ce que**
l'au moins capteur (10) comprend un accéléromètre ou un gyroscope.

12. Pièce d'instrument (1) médical ou dentaire selon l'une quelconque des revendications précédentes, **caractérisée en ce que**
l'au moins un capteur (10) comporte un capteur de température, destiné à mesurer la température d'au moins un composant de la pièce d'instrument (1) médical ou dentaire ou d'au moins un composant fixé sur la pièce d'instrument (1) médical ou dentaire ou d'une pièce d'oeuvre traitée par la pièce d'instrument (1) médical ou dentaire.

13. Dispositif de traitement (11) médical ou dentaire, **caractérisée par**
une première pièce d'instrument (1) médical ou dentaire selon l'une quelconque des revendications précédentes,
une source d'énergie (12) électrique commune, destinée à alimenter la pièce d'instrument (1) médical ou dentaire en énergie électrique et
une unité de commande, de réglage ou d'alimentation (4), destinée à réceptionner le signal de capteur de l'au moins un capteur (10).

14. Dispositif de traitement (11) médical ou dentaire selon la revendication 13, **caractérisé par** une deuxième pièce d'instrument (1C, 1D) médical ou dentaire, la première pièce d'instrument (1) médical ou dentaire et le deuxième pièce d'instrument (1C, 1D) médical et dentaire étant susceptibles d'être connectées l'une à l'autre, pour être connectées par l'intermédiaire d'un flexible d'alimentation (15) commun avec la source d'énergie (12) électrique commune et avec une unité de commande, de réglage ou d'alimentation (4), la première et la deuxième conduites électriques (6A, 6B) communes des trois conduites électriques (6A, 6B, 6C) transmettant de l'énergie électrique à partir de la source d'énergie (12) électrique commune au dispositif (2) émettant un rayonnement électromagnétique, pour le fonctionnement du dispositif (2) émettant un rayonnement électromagnétique et au système de commande (5B) de pièce d'instrument médical ou dentaire de la première pièce d'instrument (1) médical ou dentaire, pour le fonctionnement de celle-ci, et la troisième conduite électrique (6C) des trois conduites électriques (6A, 6B, 6C) étant connectée avec l'au moins un capteur (10), de telle sorte que les signaux de capteur de l'au moins un capteur (10) soient transmissibles par l'intermédiaire de la troisième conduite électrique (6C) à l' unité de commande, de réglage ou d'alimentation (4) commune.

15. Dispositif de traitement (11) médical ou dentaire selon la revendication 13 ou 14, **caractérisé en ce que**
la source d'énergie (12) électrique commune comporte une source de tension constante ou la source d'énergie (12) électrique commune est conçue pour mettre à disposition un courant continu.
